(19) Europäisches Patentamt
European Patent Office
Office européen des brevets

(11) EP 2 556 080 B1

(12) **FASCICULE DE BREVET EUROPEEN**

(45) Date de publication et mention de la délivrance du brevet:
**03.06.2015 Bulletin 2015/23**

(21) Numéro de dépôt: **11720876.9**

(22) Date de dépôt: **14.04.2011**

(51) Int Cl.:
***C07H 15/203*** *(2006.01)* ***A61K 8/60*** *(2006.01)*
***A61Q 19/08*** *(2006.01)* ***A61K 31/7034*** *(2006.01)*

(86) Numéro de dépôt international:
**PCT/IB2011/051628**

(87) Numéro de publication internationale:
**WO 2011/125057 (13.10.2011 Gazette 2011/41)**

(54) **COMPOSITION COSMETIQUE**

KOSMETISCHE ZUSAMMENSETZUNG

COSMETIC COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.04.2010 FR 1052719**

(43) Date de publication de la demande:
**13.02.2013 Bulletin 2013/07**

(73) Titulaires:
- **Guerlain**
  **75008 Paris (FR)**
- **Université de Strasbourg**
  **67000 Strasbourg (FR)**
- **Centre National de la Recherche Scientifique**
  **75016 Paris (FR)**

(72) Inventeurs:
- **SIMMLER, Charlotte**
  **F-67400 Illkirch (FR)**
- **LEPLANQUAIS, Virginie**
  **F-45450 Fay-aux-Loges (FR)**
- **NOBLESSE, Emmanuelle**
  **F-45450 Donnery (FR)**
- **ANDRE, Patrice**
  **F-45170 Neuville Aux Bois (FR)**
- **LOBSTEIN, Annelise**
  **F-67640 Lipsheim (FR)**

(74) Mandataire: **Chantraine, Sylvie Hélène et al**
**Cabinet Beau de Loménie**
**158, rue de l'Université**
**75340 Paris Cedex 07 (FR)**

(56) Documents cités:
**FR-A1- 2 924 348**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 556 080 B1

Printed by Jouve, 75001 PARIS (FR)

## Description

L'invention concerne de nouveaux dérivés de l'acide eucomique (acide 2-(4-hydroxybenzyl)-malique), des compositions cosmétiques ou dermatologiques comprenant au moins un de ces composés et des méthodes de soin utilisant lesdites compositions.

ETAT DE LA TECHNIQUE

Les mitochondries constituent l'usine de production d'énergie cellulaire, grâce à leur chaîne respiratoire et la phosphorylation oxydative. Lors du vieillissement cutané, notamment photo-induit, on observe une baisse des fonctions mitochondriales.

En règle générale, une chaîne respiratoire mitochondriale défectueuse conduit à une augmentation intracellulaire d'espèces oxygénées réactives (EOR), ce qui accélère le processus de vieillissement cutané. Cette dysfonction mitochondriale est responsable d'une baisse du métabolisme énergétique cellulaire qui compromet au final la production d'énergie cellulaire. En cosmétique, les actifs capables de stimuler le fonctionnement de la chaîne respiratoire mitochondriale, et ainsi d'activer le métabolisme énergétique cellulaire, revendiquent des fonctions anti-âges (CoQ10, Resvératrol). Par ailleurs, les actifs cosmétiques qui stimulent le renouvellement et donc la différenciation cellulaire participent au maintien d'une peau saine et jeune.

La présente invention porte sur de nouveaux composés dérivés de l'acide eucomique (acide 2-(4-hydroxybenzyl)-malique), notamment obtenus à partir d'un extrait de l'orchidée *Papilionanthe teres,* qui présentent de telles propriétés.

L'acide eucomique est connu. Il a tout d'abord été isolé à partir d'un extrait de bulbes *d'Eucomis punctata* (famille des Liliacées) (Heller et al., Helvetica Chimica Acta, 1984, 57 (6), 1766-84*),* puis identifié ensuite dans d'autres espèces végétales telles que *Lotus japonicus* (Fabaceae), *Crotalaria sessiflora* (Fabaceae), *Crataegus pinnatifada* (Rosaceae), ou bien encore *Encyclia michuacana* (Orchidaceae).

La famille des Orchidaceae (Orchidacées ou Orchidées) est une très grande famille de plantes monocotylédones. C'est la famille végétale la plus diversifiée, comptant plus de vingt-cinq mille espèces, réparties en huit-cent cinquante genres. Un certain nombre d'orchidées ont déjà fait l'objet d'études dans le domaine de la cosmétique, visant des applications pour les soins de la peau.

Par exemple, la demande de brevet FR 2 924 348 divulgue un extrait obtenu à partir d'au moins une partie de l'orchidée *Vanda teres,* maintenant classée dans le genre *Papilionanthe* en tant qu'espèce dénommée *Papilionanthe teres,* ainsi que l'utilisation dudit extrait en tant qu'agent actif dans une composition cosmétique comprenant au moins un excipient cosmétiquement acceptable, et destinée à être appliquée sur la peau pour atténuer ou retarder les effets du vieillissement de la peau.

Cet extrait de *Papilionanthe teres* est donc particulièrement intéressant du point de vue de ses activités, notamment cosmétiques ou dermatologiques. Cependant, à la connaissance des inventeurs, aucune molécule responsable de ces activités n'a été, à ce jour, ni isolée, ni identifiée.

BUTS DE L'INVENTION

La présente invention a pour but principal de fournir de nouveaux composés chimiques utilisables comme agents actifs cosmétiques ou dermatologiques aptes à atténuer ou retarder les effets du vieillissement cutané, et/ou à maintenir ou améliorer l'hydratation cutanée et/ou favoriser la cicatrisation cutanée.

Elle a également pour but de fournir ces nouveaux composés sous une forme utilisable en tant qu'agent actif dans une composition cosmétique ou dermatologique, notamment sous la forme d'extraits végétaux enrichis en au moins un de ces nouveaux composés.

Selon un autre aspect, l'invention concerne une composition cosmétique ou dermatologique contenant au moins un de ces composés en tant qu'agent cosmétique ou dermatologique, ou un tel agent actif sous forme d'extrait végétal, dans un véhicule cosmétiquement ou dermatologiquement acceptable, compatible avec une application topique sur la peau.

L'invention a également pour but de résoudre le problème technique consistant en isolement et la caractérisation d'une ou plusieurs molécules actives d'un extrait de l'orchidée *Papilionanthe teres.*

L'invention a enfin pour but de proposer une solution à ces problèmes techniques de manière particulièrement simple, relativement peu coûteuse, et utilisable à l'échelle industrielle et cosmétique.

DESCRIPTION DETAILLEE DE L'INVENTION

Les inventeurs de la présente invention ont isolé et identifié de nouveaux composés dérivés de l'acide eucomique, obtenus notamment à partir d'un extrait de l'orchidée *Papilionanthe teres.* Ces nouveaux composés sont collectivement dénommés « vandatérosides » dans la présente demande (en abrégé « VT »).

Les inventeurs ont également mis en évidence l'impact des vandatérosides de l'invention sur le fonctionnement de la chaîne respiratoire mitochondriale des kératinocytes humains (KHN et HaCaT), en s'intéressant plus précisément aux complexes I et II de la chaîne respiratoire ainsi qu'à la cytochrome C oxydase. Ils ont également montré qu'ils stimulent significativement le fonctionnement de la chaîne respiratoire mitochondriale et la différenciation cellulaire des kératinocytes humains. Ces molécules agissent donc au coeur de la régulation du métabolisme énergétique et de la régulation du stress oxydant cellulaire et présentent donc un intérêt inattendu en tant qu'agents actifs dans des compositions cosmétiques ou dermatologiques pour lutter contre le vieillissement cutané. En stimulant le renouvellement et la différenciation cellulaire, les composés participent au métabolisme cellulaire et stimulent le renouvellement du *stratum corneum* et contribuent au maintien d'une peau saine et jeune.

Les techniques employées font appel d'une part à la mesure de la transformation du XTT (2,3-bis (2-methoxy-4-nitro-5-sulfophenyl)-5-[(phenylamino) carbonyl]-2H-tetrazolium hydroxide) en formazan soluble par les complexes I et II des KHN, d'autre part à la mesure propre de l'activité enzymatique du cytochrome C oxydase extraite des mitochondries de kératinocytes humains modifiés (HaCaT).

De même, les inventeurs ont évalué l'action de ces vandatérosides sur la production de protéines d'expression (transglutaminase, involucrine, desmogléine I) de la différenciation des KHN par immuno-marquage. Ce processus de différenciation participe au renouvellement cellulaire de l'enveloppe cornéenne.

Un premier objet de l'invention vise des composés qui présentent les formules ci-dessous :

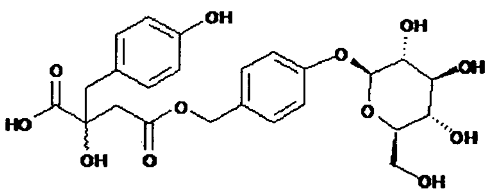

(VT1)

et en particulier le 4-(4-β-D-glucopyranosyloxybenzyl)-(2R)-2-(*p*-hydroxybenzyl)-malate,

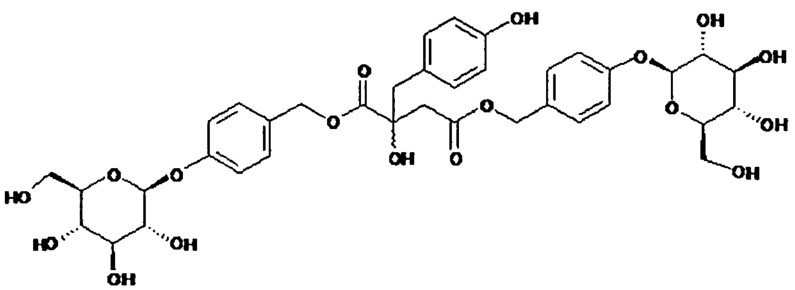

(VT2)

et en particulier le 1,4-bis(4-β-D-glucopyranosyloxybenzyl)-(2R)-2-(*p*-hydroxybenzyl)-malate,

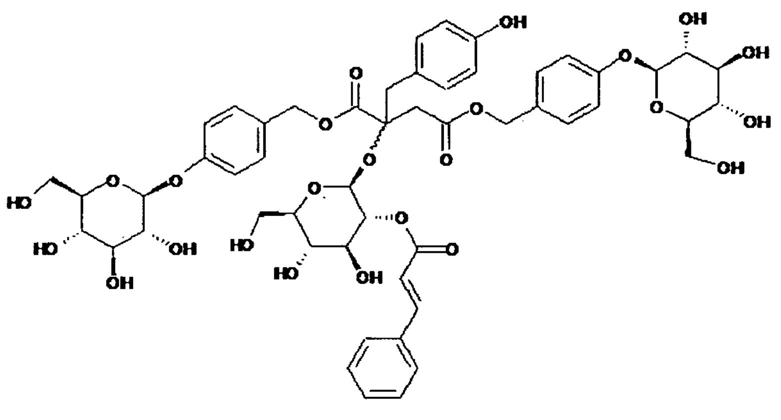

(VT3)

et en particulier le 1,4-bis(4-β-D-glucopyranosyloxybenzyl)-(2R)-2-(2-β-D-glucopyranosyl-3-trans-cinnamoyl ester)-2-(p-hydroxybenzyl)-malate.

Un deuxième objet de l'invention concerne un extrait de plante caractérisé en ce qu'il est spécifiquement enrichi en un ou plusieurs des composés tels que définis ci-dessus.

Ledit extrait est avantageusement obtenu à partir d'au moins une partie d'une orchidée, en particulier d'une orchidée appartenant au genre *Papilionanthe, Vanda* ou *Encyclia.*

Un extrait préféré est en particulier caractérisé en ce qu'il est obtenu à partir de l'orchidée *Papilionanthe teres,* notamment à partir de tiges, de racines, de fleurs de ladite orchidée, et en ce qu'il comprend au moins un composé VT1 et/ou VT2 et/ou VT3.

On entend par « spécifiquement enrichi » ou « enrichi » un extrait qui est obtenu par une étape visant à augmenter volontairement la teneur de l'extrait en composés VT1 et/ou VT2 et/ou VT3.

Ledit extrait enrichi est en particulier avantageusement obtenu par une étape d'élimination des sucres ou par fractionnement, et par exemple par chromatographie d'exclusion stérique.

Des extraits typiques sont ceux dont l'extrait sec comprend une concentration en composés VT1 et/ou VT2 et/ou VT3, supérieure à celle d'un extrait sec obtenu après extraction, sans étape visant à augmenter spécifiquement la concentration des composés précités.

De préférence ledit extrait enrichi comprend au moins 40%, au moins 50%, au moins 60%, au moins 70% et encore de préférence au moins 80% en poids de composé VT1 et/ou VT2 et/ou VT3, par rapport au poids total de l'extrait sec.

Un extrait particulier de l'invention, avantageusement préparé à partir de l'orchidée *Papilionanthe teres,* est caractérisé en ce qu'il comprend un ou plusieurs composés choisis parmi VT1 et/ou VT2 à une teneur cumulée supérieure à 40% en poids par rapport au poids de l'extrait sec, de préférence supérieure à 60%, et encore de préférence supérieure à 80%.

Un autre extrait de l'invention, également avantageusement préparé à partir de l'orchidée *Papilionanthe teres,* est caractérisé en ce qu'il comprend le composé VT3 à une teneur supérieure à 8% en poids par rapport au poids de l'extrait sec, de préférence supérieure à 15% en poids, de préférence supérieure à 25% en poids, et encore de préférence supérieure à 40% en poids.

Avantageusement l'extrait de l'invention est caractérisé en ce qu'il est constitué par au moins 80% en poids des composés VT1, VT2 et VT3 par rapport au poids total de l'extrait sec, de préférence au moins 90% en poids.

Un extrait enrichi préféré est plus particulièrement préparé à partir des tiges ou des racines de l'orchidée *Papilionanthe teres.*

Un troisième objet de l'invention porte sur une composition cosmétique ou dermatologique comprenant à titre d'agent actif cosmétique ou dermatologique, au moins un composé défini ci-dessus et/ou un extrait de plante comprenant un tel composé, avantageusement selon l'une quelconque des variantes et modes de réalisation décrits précédemment.

Outre au moins un des composés ou extraits précités, la composition cosmétique peut également comprendre un ou plusieurs autres agents actifs cosmétiques ou dermatologiques, sous forme de molécules purifiées et/ou d'extraits végétaux, présentant des effets cosmétiques similaires et/ou complémentaires à celles des desdits composés.

La composition peut ainsi notamment comprendre un ou plusieurs autres extraits de plantes, obtenus à partir de plantes entières ou de parties de plantes ou encore des solutions réalisées à partir d'extraits de ces plantes, les extraits étant avantageusement obtenus par les procédés classiquement utilisés par l'homme de l'art, et plus particulièrement par extraction à l'aide d'un solvant polaire ou d'un mélange de solvants polaires, avantageusement choisis parmi l'eau, des alcools en $C_1$-$C_4$ ou des glycols.

Les autres agents actifs cosmétiques ou dermatologiques peuvent être choisis par exemple parmi des substances ayant une activité anti-vieillissement ; des substances ayant une activité dépigmentante ou une activité éclaircissante de la peau; des substances ayant une activité amincissante ; des substances ayant une activité hydratante ; des substances ayant une activité calmante, apaisante ou relaxante ; des substances stimulant la microcirculation cutanée pour améliorer l'éclat du teint, en particulier du visage; des substances ayant une activité sébo-régulatrice pour le soin des peaux grasses; des substances destinées à nettoyer ou purifier la peau ; des substances ayant une activité anti-radicalaire.

La composition peut avantageusement comprendre au moins un extrait d'orchidée telle qu'une orchidée appartenant au genre Brassocattleya, par exemple un extrait de l'orchidée *Brassocattleya marcella,* ou au genre *Encyclia,* par exemple un extrait de l'orchidée *Encyclia michuacana* ou au genre *Cattleya,* ou au genre *Vanda,* par exemple un extrait d'une orchidée parmi *Vanda coerulea* ou *Vanda denisoniana,* ou bien encore au genre *Papilionanthe.*

Avantageusement, la composition comprend en outre au moins un excipient cosmétiquement ou dermatologiquement acceptable qui peut être notamment choisi parmi des pigments, des colorants, des polymères, des agents tensioactifs, des agents de rhéologie, des parfums, des électrolytes, des ajusteurs de pH, des agents anti-oxydants, des conservateurs, et leurs mélanges.

La composition cosmétique ou dermatologique se présente avantageusement sous forme d'un sérum, d'une lotion, d'une crème ou d'un hydrogel, notamment un masque, ou encore sous la forme d'un stick ou d'un patch.

Un quatrième objet de l'invention concerne au moins un composé tel que défini précédemment ou d'au moins un extrait de plante comprenant un tel composé, avantageusement selon l'une quelconque des variantes et modes de réalisation décrits précédemment, pour son utilisation en tant qu'agent actif cosmétique ou dermatologique dans une composition cosmétique ou dermatologique.

Avantageusement ledit composé ou ledit extrait est un agent actif destiné à lutter contre le vieillissement cutané, notamment pour atténuer ou retarder les effets du vieillissement de la peau, restructurer l'épiderme, raffermir la peau, et/ou favoriser l'atténuation ou la résorption des rides et les propriétés protectrices de l'épiderme, et/ou à maintenir ou améliorer l'hydratation cutanée et/ou à favoriser la cicatrisation cutanée.

L'invention concerne également au moins un composé tel que défini précédemment, ou un extrait de plante comprenant un tel composé, avantageusement selon l'une quelconque des variantes et modes de réalisation décrits précédemment, pour son utilisation en tant qu'agent actif dans une composition cosmétique ou dermatologique, ledit agent actif stimulant l'expression et/ou l'activité des déshydrogénases mitochondriales, et/ou de la cytochrome C oxydase, et/ou de la chaîne respiratoire des mitochondries, et/ou le métabolisme énergétique cellulaire et/ou la différentiation et le renouvellement des cellules épithéliales.

Avantageusement ledit agent actif permet d'agir au niveau de l'épiderme et en particulier des cellules épithéliales, et encore plus particulièrement des kératinocytes.

L'invention concerne également au moins un extrait de l'orchidée *Papilionanthe teres,* en particulier de tiges ou de racines de l'orchidée *Papilionanthe teres,* comprenant un tel composé, avantageusement selon l'une quelconques des variantes et modes de réalisation décrits précédemment, pour son utilisation en tant qu'agent actif destiné à lutter contre le vieillissement cutané, notamment pour atténuer ou retarder les effets du vieillissement de la peau, restructurer l'épiderme, raffermir la peau, et/ou favoriser l'atténuation ou la résorption des rides et les propriétés protectrices de l'épiderme, et/ou à maintenir ou améliorer l'hydratation cutanée et/ou à favoriser la cicatrisation cutanée., dans une composition cosmétique ou dermatologique, ledit agent actif stimulant l'expression et/ou l'activité des déshydrogénases mitochondriales, et/ou de la cytochrome C oxydase, et/ou de la chaîne respiratoire des mitochondries, et/ou le métabolisme énergétique cellulaire et/ou la différentiation et le renouvellement des cellules épithéliales.

Avantageusement les composés de l'invention sont actifs pour stimuler l'expression des protéines de différentiation des kératinocytes.

Pour chacun des objets l'invention précitée, la concentration en composé tel que défini précédemment ou en extrait comprenant un tel composé, utilisé en tant qu'agent actif cosmétique ou dermatologique, dans une composition cosmétique ou dermatologique est comprise entre 0,0001 et 1% en poids, plus particulièrement entre 0,001 et 0,1 % en poids, encore mieux entre 0,01 et 0,1 % en poids, par rapport au poids de ladite composition.

Les compositions de l'invention présentent un effet particulièrement recherché pour lutter contre le vieillissement cutané, notamment pour atténuer ou retarder les effets du vieillissement de la peau, restructurer l'épiderme, raffermir la peau, et/ou favoriser l'atténuation ou la résorption des rides et les propriétés protectrices de l'épiderme, et/ou pour maintenir ou améliorer l'hydratation cutanée et/ou pour favoriser la cicatrisation cutanée.

L'invention décrit également une méthode de soin cosmétique ou dermatologique caractérisée en ce qu'elle comprend l'application sur les zones de peau concernées, d'une composition cosmétique ou dermatologique telle que définie précédemment, en une quantité efficace pour lutter contre le vieillissement cutané, notamment pour atténuer ou retarder les effets du vieillissement de la peau, restructurer l'épiderme, raffermir la peau, favoriser l'atténuation ou la résorption des rides et les propriétés protectrices de l'épiderme, et/ou pour maintenir ou améliorer l'hydratation cutanée et/ou pour favoriser la cicatrisation cutanée.

Avantageusement, ladite méthode comprend l'application de l'adite composition sur une zone de peau du visage, du cou ou du corps, notamment des mains, présentant des signes de vieillissement tels que la présence de rides ou de ridules.

La présente invention concerne également un procédé de préparation d'un composé selon l'invention, ou d'un extrait enrichi selon l'invention, ledit procédé comprenant une étape d'extraction d'au moins une partie d'une plante, avantageusement d'une orchidée, en particulier de l'orchidée *Papilionanthe teres,* comprenant un tel composé, et au moins une étape d'enrichissement de l'extrait obtenu à l'étape d'extraction, en un ou plusieurs composés VT1 et/ou VT2 et/ou VT3.

De préférence, préalablement à l'étape d'extraction, ce procédé comprend une étape de broyage de la plante entière ou d'au moins une partie de la plante, de préférence les tiges, les feuilles ou les racines, puis une étape d'extraction de cette plante ou des parties de ladite plante.

La plante ou les parties de plantes sont avantageusement séchées ou congelées avant broyage.

De préférence, l'extraction est réalisée à l'aide d'au moins un solvant polaire. Parmi les solvants polaires on peut utiliser un solvant unique ou un mélange de solvants. Les solvants polaires utilisés sont de préférence l'eau ; un alcool, de préférence en $C_1$-$C_4$, et notamment le méthanol, l'éthanol ou un propanol ; un polyol, et notamment un glycol, et par exemple le glycérol, le propylène glycol ou le butylène glycol.

Parmi les mélanges de solvants on utilise de préférence un mélange hydro-alcoolique, et de préférence un

mélange éthanol/eau, et plus particulièrement un mélange éthanol/eau dans un rapport de l'ordre de 90/10 v/v.

L'extraction peut s'effectuer de préférence dans un solvant polaire à reflux et de préférence au Soxhlet. Il est possible d'optimiser l'extraction au Soxhlet en utilisant des solvants de polarité croissante, comme par exemple avec une succession de solvants du type alcane (par exemple pentane, hexane, cyclopentane, cyclohexane, heptane, etc.), alcane halogéné (par exemple dichlorométhane), puis alcool (par exemple éthanol ou méthanol).

L'extrait peut être ensuite concentré pour éliminer le solvant ou le mélange de solvants. On peut donc obtenir un composé sec, qui peut être solubilisé ou dispersé dans un excipient cosmétiquement ou dermatologiquement acceptable pour une utilisation finale dans une composition de l'invention.

Le procédé de l'invention comprend avantageusement une étape d'enrichissement de l'extrait obtenu à l'étape d'extraction susmentionnée, en un ou plusieurs des composés tels que définis ci-dessus. Le procédé comprend en particulier une étape d'enrichissement par élimination des sucres ou par fractionnement, et par exemple par chromatographie d'exclusion stérique.

En outre, l'extrait peut être décoloré partiellement ou complètement, notamment en présence de charbon actif après avoir été mis en présence d'un solvant polaire.

On peut purifier les fractions extraites par toute méthode connue de l'homme de l'art, et par exemple par chromatographie, notamment chromatographie sur colonne de gel, et/ou chromatographie liquide haute performance (CLHP ou HPLC).

D'autres buts, caractéristiques et avantages de l'invention, apparaîtront clairement à la lumière de la description explicative qui va suivre, faite en référence à des exemples de préparation d'extraits et de tests mettant en évidence les propriétés des extraits et à un exemple de composition cosmétique utilisant de tels extraits, donnés simplement à titre d'illustration et qui ne sauraient donc en aucune façon limiter la portée de l'invention.

Dans les exemples, tous les pourcentages sont donnés en poids, la température est en degrés Celsius, la pression est la pression atmosphérique, sauf indication contraire.

Sur les figures :

La figure 1 représente les formules semi-développées des composés VT1, VT2, et VT3 de l'invention ainsi que de l'acide eucomique ;

La figure 2 représente les spectres de masse des composés VT1, VT2, et VT3 de l'invention ;

La figure 3 représente les spectres de RMN $^{1}$H des composés VT1, VT2, et VT3 de l'invention ;

La figure 4 représente les spectres de RMN $^{13}$C (DEPT135)des composés VT1, VT2, et VT3 de l'invention ;

La figure 5 représente le profil obtenu par CLHP et détection UV de l'extrait selon l'exemple 1.2 (solubilisé dans 5mg/mLMeOH) comprenant les composés VT1, VT2, et VT3 de l'invention ;

La figure 6 représente l'effet des composés VT1, VT2, et VT3 de l'invention sur l'activation des déshydrogénases mitochondriales ; Durée de traitement 48h. Témoin négatif DMSO (0,1% ; v/v). Moyennes ± EC (écart type) calculées à partir de trois expériences.

La figure 7 représente l'activité des composés VT1, VT2, et VT3 de l'invention sur l'expression de la cytochrome C oxydase ; Traitement des cellules HaCaT : 3h. Témoin négatif : cellules non traitées (DMSO (0.1% ; v/v), témoin positif: Resveratrol (11.41 μg/ml). Moyennes ± EC (écart type) obtenues sur de trois expériences indépendantes.

La figure 8 représente l'activité des composés VT1, VT2, et VT3 de l'invention sur la biogenèse mitochondriale ; Traitement des cellules HaCaT : 48h. Témoin négatif : cellules non traitées (DMSO (0.1% ; v/v), témoin positif: Resveratrol (11.41 μg/ml). Moyennes ± EC (écart type) obtenues sur de trois expériences indépendantes.

La figure 9 représente l'effet de traitement à l'aide des composés VT1, VT2, ou VT3 de l'invention sur l'expression de la transglutaminase ;

La figure 10 représente une photographie au microscope électronique de l'expression de la transglutaminase en présence des composés VT1, VT2, ou VT3 de l'invention ;

La figure 11 représente l'effet de traitement à l'aide des composés VT1, VT2, ou VT3 de l'invention sur l'expression de l'involucrine ;

La figure 12 représente l'effet de traitement à l'aide des composés VT1, VT2, ou VT3 de l'invention sur l'expression de la desmogléine 1 ;

La figure 13 représente une photographie au microscope électronique de l'expression de la desmogléine 1 en présence des composés VT1, VT2, ou VT3 de l'invention;

## EXEMPLE

### Exemple 1 : Extraits de *Papilionanthe teres.*

Différentes parties de l'orchidée *Papilionanthe teres* sont analysées afin de détecter la présence de vandatérosides dans chacune de ces parties de la plante, et d'en déterminer la teneur.

100 g de matière végétale (MV) sèche, constituée des tiges (exemple 1.1 et 1.2), de racines (exemple 1.3) ou de feuilles (exemple 1.4) de l'orchidée *Papilionanthe teres* (origine Tian Zi, Chine), sont broyés avant l'étape d'extraction.

Exemple 1.1. Extrait hydro-alcoolique de tiges de *Papilionanthe teres*

Un extrait total hydroalcoolique de la MV sèche (éthanol (EtOH)/eau 90/10 v/v, rapport MV/solvant: 1/15) est réalisé à reflux (30min, 80°C). Cet extrait total est obtenu avec un rendement massique de 12,8% (masse d'extrait/masse de MV sèche).

Exemple 1.2. Extrait méthanolique de tiges de *Papilionanthe teres*

Une extraction de type Soxhlet (appareillage SOXTEC Avanti 2055), plus spécifique que celle de l'exemple 1.1, permet d'épuiser la MV sèche par l'utilisation successive de trois solvants de polarité croissante: cyclohexane (45min, 180°C), dichlorométhane (cycle 1 : 45min, 180°C, cycle 2 : puis 30min, 180°C) et méthanol (MeOH) (2 cycles de 45min, 180°C).

Cet extrait méthanolique, qui concentre les vandatérosides, est obtenu avec un rendement massique de 6,9% (masse d'extrait/masse de MV sèche).

Exemple 1.3. Extrait hydro-alcoolique de racines de *Papilionanthe teres*

Un extrait hydro-alcoolique total est réalisé selon les conditions suivantes : EtOH/eau 90/10, en utilisant un rapport MV /solvant de 1/15. L'extrait est réalisé à reflux (30 minutes, 80°C). Le rendement massique obtenu pour l'extrait de racines est de 14,8%.

Exemple 1.4. Extrait hydro-alcoolique de feuilles de *Papilionanthe teres*

Un extrait hydro-alcoolique total est réalisé selon les conditions suivantes : EtOH/eau 90/10, en utilisant un rapport MV sèche broyée/solvant de 1/15. L'extrait est réalisé à reflux (30 minutes, 80°C). Le rendement massique obtenu est de 15,0% pour l'extrait de feuilles.

Ces extraits (exemples 1.1 à 1.4) sont analysés selon les conditions décrites dans l'exemple 2 (Fractionnement, isolement) pour confirmer la présence de 3 composés respectivement dénommés VT1, VT2 et VT3 après isolement et élucidation structurale. Leurs concentrations respectives dans les différents extraits et fractions est déterminée par RP-HPLC selon la méthode de l'exemple 2.1, par étalonnage externe : des dilutions en série de chacun des composés isolés VT1, VT2 et VT3 sont réalisées pour déterminer la courbe d'étalonnage.

Les résultats sont indiqués dans le tableau 1 ci-dessous (voir aussi fig.5):

Tableau 1

| | VT1 | VT2 | VT3 |
|---|---|---|---|
| Extrait hydro-alcoolique de tiges de *P. teres* | 6,7 ± 1,7 | 14,1 ± 0,6 | 2,6 ± 0,2 |
| Extrait MeOH de tiges de *P. teres* | 9,4 ± 0,4 | 19,3 ± 1,2 | 3,4 ± 0,7 |
| Extrait hydro-alcoolique de feuilles de *P. teres* | 8,1 ± 0,6 | 13,4 ± 0,9 | 2,9 ± 0,0 |
| Extrait hydro-alcoolique de racines de *P. teres* | 1,6 ± 0,4 | 24,8 ± 2,5 | 2,0 ± 0,0 |
| *Tableau 1: concentrations des vandatérosides VT1, VT2 et VT3 dans les différents extraits de P. teres (% m/m moyennes ± EC, injections en triplicata)* | | | |

CONCLUSIONS

Il ressort de ces analyses que :

- les vandatérosides VT1, VT2 et VT3 sont présents dans toutes les parties de plantes de *Papilionanthe teres* (tiges, feuilles et racines).
- l'extrait de racines de *Papilionanthe teres* concentre VT2 à une teneur égale à 1,8 fois celle déterminée dans les extraits hydro-alcooliques de tiges ou de feuilles.

**Exemple 2** : **Extraits spécifiquement enrichis en vandatérosides.**

On sélectionne les extraits obtenus selon l'exemple 1 ci-dessus pour procéder à une étape de fractionnement stérique visant à enrichir spécifiquement ces extraits en composés respectivement dénommés VT1, VT2 et VT3 après isolement et élucidation structurale.

Exemple 2.1. Fractionnement par Chromatographie sur colonne de gel

Le fractionnement de l'extrait méthanolique obtenu selon l'exemple 1.2. est réalisé sur colonne de gel Sephadex LH 20 (LH20-100-Sigma) (28*180 mm) à l'aide d'un gradient de solvant polaire (passage de 100 % d'eau à 100% de méthanol, débit de 1ml/min).

1 gramme (g) de cet extrait méthanolique est dilué dans un mélange eau/méthanol (50/50 v/v) et déposé sur 30 g de gel Sephadex. Les molécules polaires sont séparées selon leur taille et leur solubilité dans le solvant d'élution.

On obtient 20 fractions de 10ml qui sont rassemblées selon leurs profils similaires obtenus par chromatographie sur couche mince (Eluant: acétate d'éthyle: acide acétique: acide formique : eau (100:11:11:26 v/v/v/v, Support : gel de silice 60 $F_{254}$ (0.25mm, MERCK), Révélation : Vanilline (Merck) -$H_2SO_4$ (97% Flucka) 5%) pour donner au final dix fractions.

Des analyses en Chromatographie Liquide Haute Performance en phase inverse (RP-HPLC) visent à identifier les fractions, parmi les dix de départ, qui concentrent les composés présents dans l'extrait total.

L'analyse en RP-HPLC est conduite à l'aide d'un chromatographe (Varian), muni d'une colonne Nucleodur $C_{18}$ec (5 μm, 250 mm x 4.6 mm i.d.), équipé d'une pompe (Prostar 230) et d'un détecteur à barrettes de diodes (Prostar 330). On utilise à titre de solvant d'élution un mélange MeOH-(eau + 0,1% d'acide formique ($HCO_2H$)), à un débit 1 ml/min, selon un gradient linéaire partant de 20% jusqu'à 47% MeOH en 13,5 minutes, puis en maintenant 47% MeOH pendant 10 minutes et enfin de 47% à 100% MeOH en 10 minutes. Les fractions sont visualisées à une longueur d'onde comprise entre 200 nm et 400 nm.

On recueille une masse de 178 mg de la fraction A et de 39,5 mg de la fraction B.

Exemple 2.2. Isolement des composés:

On utilise les deux fractions A et B sélectionnées à l'exemple 2.1, pour purifier les composés de l'invention, et procéder à leur quantification dans chacune des fraction de l'exemple 2.1.

L'isolement des composés VT1, VT2 et VT3 à partir de chacune des deux fractions sélectionnées, est réalisé par CLHP semi-préparative, à l'aide d'un chromatographe Gilson, équipé d'une pompe à gradient (Gilson 322), d'un détecteur UV (Gilson UV-VIS 151) et d'une colonne Nucleodur C18ec (5 μm, 250 mm x 21 mm i.d.). Chaque fraction est préparée à 30mg/ml dans du MeOH puis filtrée (Filtres Minisart RC 15, 0,45μm). Les conditions d'élution font appel à un mélange MeOH-(eau + 0,1% $HCO_2H$ dans les conditions suivantes: de 20% à 47% MeOH en 13,5 minutes, 47% MeOH pendant 10 minutes et retour à 20% en 5 minutes au débit de 14 ml/min. La détection se fait à 205 nm.

Après purification par CLHP semi-préparative de la fraction A,, on recueille une masse de 64mg du composé VT1 et une masse de 112 mg du composé VT2.

Après purification de la fraction B selon la même méthode, on recueille une masse de 20mg du composé VT3.

Les teneurs en chacun des composés VT1, VT2, VT3 et la teneur cumulée (VT1 +VT2+VT3) de chacune des fractions A et B et dans la fraction (A+B), sont indiquées dans le tableau 2 ci-dessous.

Tableau 2

| | VT1 | VT2 | VT3 | VT1+VT2+VT3 |
|---|---|---|---|---|
| %VT dans la fraction A+B | | | | 90,00 |
| %VT dans la fraction A | 35,00 | 63,00 | - | 98,00 |
| % VT dans la fraction B | - | - | 51,00 | 51,00 |

CONCLUSIONS

Les fractions A et B constituent chacune un extrait spécifiquement enrichi en composés qui constitue un objet de l'invention, puisque chacune est constituée à plus de 50% en poids de composés).

Ces deux fractions peuvent également être assemblées en une unique fraction (A+B) qui constitue également un exemple d'extrait spécifiquement enrichi de l'invention. Cet assemblage permet d'obtenir un extrait comprenant

chacun des trois composés identifiés (VT1, VT2 et VT3), le tout se trouvant à une teneur de 90% en poids dans l'extrait sec.
Chacun de ces extraits est ainsi utilisable en tant qu'agent actif cosmétique ou dermatologique dans des compositions cosmétiques ou dermatologiques.

**Exemple 3 : élucidation structurale des composés**

L'élucidation structurale des composés extraits de la MV de l'orchidée *Papilionanthe teres* (exemple 1.2), puis isolés par fractionnement stérique et R-CLHP et fait appel à un ensemble de techniques spectrales.

**• Materiel**

Spectrométrie de Masse (SM)

Les vandaterosides sont analysés par chromatographie liquide haute performance Agilent 1200RRLC, équipée d'une colonne $C_{18}$ Supelco Discovery (25cm x 2,1mm i.d. x 5μm) et couplée à un spectromètre de masse Agilent 6520 Accurate Mass QTOF muni d'une source ESI.
Les conditions d'élution font appel à un mélange Acétonitrile (ACN)-(eau + 0,01% $HCO_2H$) dans les conditions suivantes: de 2% à 50% ACN en 30 minutes, puis passage à 95% d'ACN en 5 minutes, maintien à 95% d'ACN pendant 5 minutes et retour à 2% d'ACN en 5 minutes, avec un débit de 0,6 ml/min.
Par ailleurs, les spectres de masse haute résolution de type ESI MS ont été obtenus sur un spectromètre Bruker microToF-Q avec une source ESI positive. Les calibrations ont été réalisées à l'aide d'une solution à $10^{-4}$M de formate de Li dans un mélange isopropanol/eau (50/50 v/v).

Résonnance Magnétique Nucléaire (RMN)

Les analyses RMN ont été réalisées sur un spectromètre Bruker 400 MHz Avance III. L'obtention de spectres 1D ($^1$H et $^{13}$C) et 2D (Cosy, HSQC, HMBC) est nécessaire pour une élucidation structurale complète des molécules isolées. Les spectres sont analysés sur logiciel RMN Notebook.
Un minimum de 10 mg des molécules purifiées est dilué dans 600μl du DMSO deutéré (DMSO $D_6$, $C_2D_6OS$; 99.9 %D, Sigma). La concentration minimale ainsi obtenue est voisine de 16mg/ml.

Spectrométrie Ultra Violet (UV)

Les spectres UV caractéristiques des vandatérosides sont obtenus à l'aide d'un appareillage Shimazu UV-2401PC. Les molécules sont dissoutes dans du MeOH à $5.10^{-5}$M pour VT1 et $2,5.10^{-5}$M pour VT2 et VT3. Le coefficient d'extinction molaire ξ ($mor^1.L.cm^{-1}$) est déterminé selon la loi de Beer-Lambert :

$$A= \xi \times c \times l$$

A= Absorbance de la molécule à une longueur d'onde λ
C= concentration de la molécule (en mol/L)
I= longueur du trajet optique (en cm)

Polarimétrie

La détermination de pouvoir rotatoire [α] spécifique de la lumière polarisée par le carbone asymétrique des vandatérosides est réalisée sur un polarimètre Perkin Elmer 341. Les molécules sont maintenues en solutions à 1mg/ml dans du MeOH et placées dans la cellule de mesure du polarimètre afin de mesurer l'angle de rotation de la lumière polarisée à 589nm et 20°C. On détermine ainsi $[\alpha]^{20°}$ de chacune des molécules selon la loi de Biot :

$$a= [a]^{T°} \times l \times c$$

a = angle de rotation observé (en degrés)
l = longueur de la cuve (en dm)
c = concentration de la solution (en g/ml)

[α] $^{T°}$=pouvoir rotatoire spécifique défini à une température T et mesuré pour une longueur d'onde donnée (exprimé en $g^{-1}.mL.dm^{-1}$)

**RESULTATS DE L'ELUCIDATION STRUCTURALE**

Les données du spectre RMN obtenu pour chacun des composés VT1, VT2 et VT3, sont indiquées dans le tableau 3 ci-dessous :

Tableau 3

| | VT1 | | VT2 | | VT3 | |
|---|---|---|---|---|---|---|
| **N°** | **$^{13}$C** | **$^{1}$H (*J* in Hz** | **$^{13}$C** | **$^{1}$H (*J* in Hz)** | **$^{13}$C** | **$^{1}$H (*J* in Hz)** |
| 1 | 173.73 | | 173.42 | | 170.00 | |
| 2 | 75.30 | | 75.31 | | 80.36 | |
| 3 | 42.45 | 2.41 (d: 16.0)<br>2.74 (d: 16.0) | 42.35 | 2.48 (d: 16.7)<br>2.84 (d: 16.7) | 39.51 | 2.95 (d: 17.7)<br>2.81 (d: 17.7) |
| 4 | 171.64 | | 169.42 | | 169.00 | |
| 5 | 43.64 | 2.77 (m) | 43.53 | 2.81(m) | 40.82 | 2.99 (m)<br>3.01 (m) |
| 6 | 125.70 | | 125.43 | | 125.34 | 6.21 (d, 2.5) |
| 7,11 | 131.50 | 6.90 (d: 8.5) | 131.17 | 6.91 (d: 8.5) | 131.47 | 6.96 (d: 8.5) |
| 8, 10 | 114.91 | 6.59 (d: 8.5) | 114.48 | 6.60 (d: 8.5) | 114.43 | 6.58 (d: 8.5) |
| 9 | 155.96 | | 155.90 | | 156.11 | |
| 1' | 129.14 | | 128.98 | | 130.35 | |
| 7' | 65.85 | 4.98 | 65.15 | 4.96 | 65.58 | 4.93 (d : 5)<br>4.97 (d : 5) |
| 2', 6' | 129.40 | 7.22 (d: 8.5) | 129.44 | 7.25 (d: 8.6) | 129.71 | 7.27 (d: 8.4) |
| 5', 3' | 116.20 | 6.98 (d: 8.5) | 115.96 | 7.01 (d: 8.6) | 116.06 | 7.03 (d: 8.4) |
| 4' | 157.53 | | 157.08 | | 157.31 | |
| 4'O-Glc-1 | 100.54 | 4.84 (d:7.0) β | 100.17 | 4.86 (d:6.8) β | 100.06 | 4.88 (d:7.2) β |
| 2 | 73.12 | 3.23 (m) | 73.05 | 3.23 (m) | 73.22 | 3.23 (m) |
| 3 | 77.00 | 3.35 (m) | 76.84 | 3.30 (m) | 76.59 | 3.28 (m) |
| 4 | 69.74 | 3.15 (m) | 69.53 | 3.15 (m) | 69.67 | 3.15 (m) |
| 5 | 76.60 | 3.28 (m) | 76.44 | 3.28 (m) | 76.98 | 3.33(m) |
| 6 | 60.70 | 3.68 (dd : 1.5/12.5)<br>3.45 (dd : 6.1/12.5) | 60.52 | 3.68 (dd: 4.9/11)<br>3.46 (dd: 5.3/11) | 60.66 | 3.69 (dd: 1.7/11.7)<br>3.47 (dd: 6.8/11.7) |
| 4"-O-Glc-1 | | | 100.17 | 4.84 (d: 6.8) β | 100.12 | 4.87 (d: 7.2) β |
| 2 | | | 73.05 | 3.23 (m) | 73.22 | 3.23 (m) |
| 3 | | | 76.84 | 3.30 (m) | 76.59 | 3.28 (m) |
| 4 | | | 69.53 | 3.15 (m) | 69.67 | 3.15 (m) |
| 5 | | | 76.44 | 3.28 (m) | 76.98 | 3.33(m) |
| 6 | | | 60.52 | 3.68 (dd: 4.9/11)<br>3.46 (dd: 5.3/11) | 60.66 | 3.69 (dd: 1.7/11.7)<br>3.47 (dd: 6.8/11.7) |
| 1 " | | | 128.86 | | 128.71 | |
| 7" | | | 65.63 | 4.96 | 66.23 | 5.01 (d : 6.6) |

(suite)

| | VT1 | | VT2 | | VT3 | |
|---|---|---|---|---|---|---|
| **N°** | **$^{13}C$** | **$^{1}H$ (*J* in Hz** | **$^{13}C$** | **$^{1}H$ (*J* in Hz)** | **$^{13}C$** | **$^{1}H$ (*J* in Hz)** |
| 2",6" | | | 129.40 | 7.22 (d:8.6) | 129.64 | 7.27 (d:8.4) |
| 5",3" | | | 115.90 | 7.00 (d:8.6) | 116.16 | 7.03 (d:8.4) |
| 4" | | | 157.08 | | 157.34 | |
| 3-O-Glc-1 | | | | | 98.04 | 4.82 (d:7.8) β |
| 2 | | | | | 71.60 | 3.26(m) |
| 3 | | | | | 77.98 | 4.92 (dd: 9.5/9.5) |
| 4 | | | | | 67.44 | 3.39(m) |
| 5 | | | | | 76.50 | 3.10(m) |
| 6 | | | | | 60.28 | 3.49 (dd : 4.5/11.8)<br>3.68 (dd : 1.7/11.8) |
| Cin-1 | | | | | 165.71 | |
| Cin-2 | | | | | 118.42 | 6.67 (d: 15.7) |
| Cin-3 | | | | | 144.19 | 7.68 (d: 15.7) |
| Cin-4 | | | | | 134.17 | |
| Cin-5,9 | | | | | 128.26 | 7.74 (dd: 2.8/6.8) |
| Cin-6,8 | | | | | 128.94 | 7.44 (m) |
| Cin-7 | | | | | 128.67 | 7.44(m) |

*Tableau 3: spectre RMN ($^{1}H$: 400MHz, $^{13}C$: 100MHZ, DMSO $D_6$, $\delta$ en ppm, J in Hz) obtenu pour chacun des composés VT1, VT2 et VT3.*

Les résultats obtenus pour les différentes méthodes analytiques mises en oeuvre sont détaillés ci-dessous pour chaque composé.

• **VT1**

Aspect : résine de couleur jaune
Solubilité : soluble dans le MeOH et le DMSO, et peu soluble dans l'eau RMN : $^{1}H$ NMR and $^{13}C$ NMR data (DMSO $D_6$, 400MHz and 100MHz)
→ **figures** 3 à 4 et tableau 3.
MS : HR-ESI-MS ion $[(M+NH4)]^+$ à *m/z* = 526.19236 (formule moléculaire calculée pour $C_{24}H_{28}O_{12}$: 508,15808 Δ: -0,89ppm). → figure 2 et tableau 4

## Tableau 4

**Peak List**

| m/z | z | Abund | Formula | Ion |
|---|---|---|---|---|
| 526.19236 | 1 | 734161 | C24 H32 N O12 | (M+NH4)+ |
| 527.19494 | 1 | 168021 | C24 H32 N O12 | (M+NH4)+ |

**Formula Calculator Results**

| Formula | Best | Mass | Tgt Mass | Diff (ppm) | Ion Species | Score |
|---|---|---|---|---|---|---|
| C24 H28 O12 | VRAI | 508,15853 | 508,15808 | -0,89 | C24 H32 N O12 | 95,88 |

UV : maxima d'absorption à 203 (log ξ 4.2), 225 (log ξ 4.0) et 278 (log ξ 3.2)nm. → **figure 5**
$[\alpha]_{MeOH}^{20°}$ = -50° $g^{-1}$.mL.$dm^{-1}$

Hydrolyse acide

Après hydrolyse acide des molécules (3h à 80°C, en présence de 2,0 M HCl), la phase aqueuse est lavée à 3 reprises à l'aide de n-butanol saturé en eau, puis évaporée à sec. Un mélange (1:4, v/v) de pyridine et 1-(trimethyl-Silyl)imidazole (Sigma) est ajouté sur le résidu sec puis chauffé pendant 1 heure à 60°C pour réaliser une dérivation. 1 à 2μl du mélange réactionnel sont ensuite dilués dans 500μl de $CH_2Cl_2$ analytique puis analysés par chromatographie gazeuse (de type Trace GS Ultra) munie d'une colonne capillaire TR-5MS SQC (15mx0,25mm * 0,25μm) et couplée à un spectromètre de masse (DSQII Thermo Scientific). La détection est obtenue par impact électronique à 70eV. Les conditions utilisées sont les suivantes : 1 minute à 40°C puis gradient de 10°C/min jusqu'à 250°C et maintien d'une phase stationnaire à 250°C (débit d'Hélium 1ml/min, température de l'injecteur 250°C, température de la ligne de transfert 285°C). La détection est réalisée après 2 minutes d'analyse, l'intervalle de mesure de masse s'échelonne de 0 à 500.

Le sucre libéré à l'issu de l'hydrolyse acide est identifié par comparaison au temps de rétention et spectre de masse du témoin D-glucose (Merck) (Tr=15,60 minutes) ayant subi le même processus de dérivation. Pour les trois vandatérosides analysés, le sucre identifié après hydrolyse est le D-glucose.

• **VT2**

Aspect : poudre blanche amorphe
Solubilité : dans l'eau, formation d'un gel à environ 10mM. VT 2 est soluble dans le MeOH et dans le DMSO, peu soluble dans l'eau.
RMN : $^1H$ NMR and $^{13}C$ NMR data (DMSO $D_6$, 400MHz and 100MHz)
→ **figures 3 à 4 et tableau 3.**
MS : HR-ESI-MS ion $[(M+NH4)]^+$ à *m/z*= 794.28867 (formule moléculaire calculée pour $C_{37}H_{44}O_{18}$: 776.25276 Δ:-0,01ppm). → **figure 2 et tableau 5**

Tableau 5

**Peak List**

| m/z | z | Abund | Formula | Ion |
|---|---|---|---|---|
| 213.09124 | | 35369 | | |
| 359.11334 | | 139175 | | |
| 453.15531 | 1 | 145631 | | |
| 454.15825 | 1 | 36147 | | |
| 465.15525 | | 43977 | | |
| 794.28867 | 1 | 618328 | C37 H48 N O18 | (M+NH4)+ |
| 795.29123 | 1 | 229976 | C37 H48 N O18 | (M+NH4)+ |
| 796.29232 | 1 | 58672 | C37 H48 N O18 | (M+NH4)+ |
| 799.242 | | 31867 | C37 H44 Na O18 | (M+Na)+ |

**Formula Calculator Results**

| Formula | Best | Mass | Tgt Mass | Diff (ppm) | Ion Species | Score |
|---|---|---|---|---|---|---|
| C37 H44 O18 | VRAI | 776,25277 | 776,25276 | -0,01 | C37 H44 Na O18 | 98,6 |

UV : maxima d'absorption à 203 (log ξ 4.5) 225 nm (log ξ 4.5) et 278 (log ξ 3.5) nm. →**figure 5**
$[\alpha]_{MeOH}{}^{20°}$ = -56° $g^{-1}.mL.dm^{-1}$

Hydrolyse acide : se référer à VT1

• **VT3**

Aspect : résine de couleur jaune
Solubilité : composé soluble dans le MeOH et le DMSO, peu soluble dans l'eau.
RMN : $^1H$ NMR and $^{13}C$ NMR data (DMSO $D_6$, 400MHz and 100MHz)
→ **figures 3 à 4 et tableau 3.**
MS : HR-ESI-MS ion $[(M+NH4)]^+$ à *m/z* = 1086.38484 (formule moléculaire calculée pour $Q_2H_{60}O_{24}$: 1068, 34745 Δ:-1,89ppm). →**figure 2 et tableau 6**

Tableau 6

Cc . 1/z)

Peak List

| m/z | z | Abund | Formula | Ion |
|---|---|---|---|---|
| 107.04922 | | 15338 | | |
| 131.04919 | | 111862 | | |
| 149.05972 | | 39325 | | |
| 293.10284 | 1 | 220337 | | |
| 294.10592 | 1 | 33624 | | |
| 359.11327 | | 35374 | | |
| 453.15517 | | 36506 | | |
| 455.15568 | | 34663 | | |
| 818.28701 | | 16049 | | |
| 855.17968 | | 30955 | | |
| 1086.38484 | 1 | 287785 | C52 H64 N O24 | (M+NH4)+ |
| 1087.38772 | 1 | 157791 | C52 H64 N O24 | (M+NH4)+ |
| 1088.38858 | 1 | 54091 | C52 H64 N O24 | (M+NH4)+ |
| 1091.33884 | 1 | 44018 | C52 H60 Na O24 | (M+Na)+ |
| 1092.34148 | 1 | 25636 | C52 H60 Na O24 | (M+Na)+ |
| 1096.31521 | 2 | 21723 | | |
| 1096.81644 | 2 | 24710 | | |
| 1097.31801 | 2 | 19284 | | |

Formula Calculator Results

| Formula | Best | Mass | Tgt Mass | Diff (ppm) | Ion Species | Score |
|---|---|---|---|---|---|---|
| C52 H60 O24 | VRAI | 1068,34948 | 1068,34745 | -1,89 | C52 H60 Na O24 | 96,2 |

UV : maxima d'absorption observés à 223 (log ξ 4.5) nm and 276 (log ξ 3.5) nm. → **figure 5**
$[\alpha]_{MeOH}^{20°}$ = -37° $g^{-1}$.mL.$dm^{-1}$

Hydrolyse acide : se référer à VT1

**Exemple 4 : activité biologique des composés de l'invention**

Traitements

On prépare une solution-mère de chacun des trois composés isolés précédemment, VT1, VT2 et VT3, dans du DMSO (Sigma).

Les solutions mères sont ensuite diluées directement dans le milieu de culture, en vue du traitement des cellules (kératinocytes humains normaux KHN ou lignée de cellules HaCaT).

• **Stimulation des déshydrogénases mitochondriales sur kératinocytes.**

Les kératinocytes humains normaux (KHN), isolés de paupières de femmes de type caucasien de 37ans, sont cultivés en passage 3 dans du Keratinocytes Serum Free Medium (KSFM, Invitrogen) complémenté (Invitrogen) et additionné de 5% de sérum de veau foetal (SVF, Invitrogen).

Les KHN sont ensemencés dans des plaques de 96 puits (Greiner Bio One) à raison de $1.10^4$ cellules/puits, puis mis à incuber à 37°C, 5% $CO_2$ pendant 24h. Ces cellules sont ensuite traitées, après irradiation aux $UV_B$ (60mJ/$cm^2$ irradiateur Vilbert-Lourmat) ou sans irradiation, par des dilutions sérielles des composés VT1, VT2 et VT3 (de 6,25 à 0,78μg/ml) dilués dans du DMSO (sigma) 0,1%v/v en concentration finale. Les plaques sont mises à incuber à 37°C, 5% $CO_2$. Les cellules non traitées contenant 0,1% v/v de DMSO sont considérées comme contrôle négatif. Après 48h de traitement, les surnageants sont éliminés et la solution de XTT (Cell proliferation kit II, Roche Diagnostic) est déposée dans chaque puits afin de déterminer l'activité globale des déshydrogénases mitochondriales. Cette activité est évaluée grâce à la transformation de sel de tetrazolium XTT en formazan soluble, grâce à un mécanisme cellulaire faisant appel aux activités enzymatiques de la glycolytique NAD (P) H déshydrogénases (complexe I) et de la succinate déshydrogénases (complexe II), constitutives de la chaîne respiratoire mitochondriale. La quantité de formazan coloré (déterminée par lecture de densité optique à 450nm) est directement reliée d'une part à l'activité enzymatique des déshydrogénases mitochondriales et d'autre part à la quantité de cellules métaboliquement actives *(*Roehm et al. J. Immunol. Methods, 1991; 142: 257-265). Après 3 heures d'incubation, le XTT est réduit en un formazan soluble par les déshydrogénases. La lecture des plaques de 96 puits se fait à 450nm avec un spectrofluoromètre (SpectraFluor Plus, Tecan). Les protéines totales sont dosées dans chaque puits par la technique du BCA (Bicinchoninic acid assay (kit Uptima Interchim).

Les résultats sont exprimés en moyenne des densités optiques (DO) à 450nm et normalisés par rapport à la concentration totale en protéines cellulaires (exprimée en μg/ml), ce qui permet de mesurer uniquement l'activité des déshydrogénases et de s'affranchir de l'impact de la prolifération cellulaire sur la mesure des DO obtenues.

Les résultats moyens obtenus pour chaque concentration de molécules (Rm) sont comparés à ceux obtenus pour le contrôle négatif (Rc) :

Rm/Rc >1 indique une stimulation de l'activité des déshydrogénases mitochondriales.

**• Stimulation de la cytochrome C oxydase sur kératinocytes humains modifiés (HaCaT)**

L'objectif de l'étude est d'évaluer le potentiel des composés objet de l'invention pour stimuler les performances de la chaîne respiratoire mitochondriale. Les essais sont menés sur des kératinocytes humain génétiquement modifiés (HaCaT).

Les cellules HaCAT ont été cultivées en plaque de 6 puits dans du milieu RPMI (Rosewell Park Memorial Institute) 1640 w/o HEPES (invitrogen) supplémenté avec 10 % de sérum de veau foetal (SVF, Invitrogen) et un mélange pénicilline/streptomycine jusqu'à confluence. Elles ont ensuite été traitées dans du DMEM (Dulbecco's Modified Eagle Medium, Invitrogen) avec 4,5 g/L de glucose, 0,1 % de BSA (Bovine Serum albumine AM2618 invitrogen) par des dilutions sérielles de vandatérosides (de 6,25 à 0,78μg/ml) dilués dans du DMSO (0,1%v/v concentration finale). Le resveratrol (Sigma) à 50μM soit 11,41μg/ml, a été utilisé comme référence positive. Les cellules non traitées contenant 0,1% v/v de DMSO ont été considérées comme contrôle négatif. Après 3h de traitement, les cellules ont été lysées (20mM HEPES, 0,1%Triton, 1mM EDTA) et le dosage des protéines cellulaire totales a été réalisé par la technique de Bradford (B6916 Sigma). La concentration en protéines totales de chaque échantillon a été ensuite ajustée à 2mg/ml.

Le lysat cellulaire a été incubé en présence d'un tampon d'essai (Sigma) afin de déterminer la cinétique enzymatique sans ajout de substrat, notée Vo. Le cytochrome C réduit (from equine heart C-7752 sigma) par du DTT (DiThioTreitol, Sigma) a été ajouté dans le tampon d'essai à 50μM afin de mesurer la vitesse de la réaction enzymatique. La disparition du cytochrome C réduit a été suivie à l'aide d'un spectrophotomètre [Beckman DU 640] à 550nm pendant 90 secondes selon les instructions du fabricant (Sigma). Les résultats ont été calculés à partir des courbes de cinétique enzymatique et sont exprimés en activité de la cytochrome C oxydase (U/μg de protéines). Les équations permettant le calcul des activités enzymatiques sont les suivantes :

- Activité enzymatique du cytochrome C en U/ml = $A_{COX}$ = (DA/min)/21,84 avec :
- DA/min= A/min(v) - A/min (v0)
- 21,84 (mM) = ε (coefficient d'extinction molaire) du ferrocytochrome C à 550nm
- A= Absorbance à 550nm
- A/min(v) = Absorbance à 550nm au temps « v » en minutes, correspondant au temps de la mesure après m'ajout du substrat ;
- A/min (v0) = Absorbance à 550nm au temps « v0 » (au départ, c'est-à-dire avant l'ajout de substrat) en minutes.

Les résultats sont exprimés par comparaison de l'activité enzymatique mesurée en présence des vandatérosides ($A_{COX}$ échantillons) par rapport à l'activité enzymatique contrôle ($A_{COX}$ contrôle) : soit ($A_{COX}$ échantillons) / ($A_{COX}$ contrôle).

Un rapport calculé ($A_{COX}$ échantillons) / ($A_{COX}$ contrôle) supérieur à 1 indique une stimulation de l'activité du cytochrome C oxydase en présence des échantillons.

**• Mesure de la biogenèse mitochondriale sur kératinocytes humains modifiés (HaCaT)**

Pour les vandatérosides VT1 et VT2, qui ont montré un effet stimulant de l'activité du cytochrome C, on réalise des mesures de biogénèse mitochondriale.

L'objectif est de vérifier si l'augmentation de l'activité enzymatique mesurée est liée ou non à une stimulation basale de la chaîne respiratoire des mitochondries initialement présentes ou à une augmentation du nombre de mitochondries intracellulaires, indicatrice d'une stimulation de la biogenèse mitochondriale.

Les cellules HaCaT sont cultivées en plaque de 6 puits puis traitées comme décrit ci-dessus. Après 48h de traitement, les cellules sont lysées à l'aide d'un tampon constitué de 10mM de Tris-base, 1mM EDTA, 0,3M d'acétate de sodium et 1% de SDS (1ml /puits) et selon une durée d'incubation d'une nuit à 55°C. L'ADN mitochondrial est extrait du lysat cellulaire avec une solution de phénol (rapport 1/1). Deux cycles de centrifugation à 4000 rpm (5 minutes) sont réalisés. Une solution de $CHCl_3$/iso-amyle alcool (24 :1,v/v) est ensuite ajoutée à la phase aqueuse supérieure, récupérée après décantation. Le mélange est ensuite centrifugé (5 minutes, 4000 rpm, 4°C).

On induit la précipitation de l'ADN total par ajout au surnageant d'un mélange éthanol absolu/acétate de sodium 3M (2.5/1.10$^{e}$,v/v). Après centrifugation (30 minutes, 12000rpm, 4°C) les culots sont lavés avec de l'EtOH à 70° v/v, puis séchés et repris dans de l'eau désionisée. Le dosage de l'ADN est réalisé à l'aide d'un spectromètre (Nanodrop Thermo Scientific). Les solutions d'ADN sont ajustées à une concentration de 10ng/μl.

L'expression de trois gènes différents est mesurée par qPCR. Cette technique permet de mesurer la quantité d'ADN polymérisé grâce à un marqueur fluorescent. Les gènes sélectionnés sont deux gènes mitochondriaux 16S et COX2, correspondant respectivement aux gènes d'expression d'un ribosome mitochondrial et du cytochrome C oxydase, ainsi qu'un gène UCP-2 localisé dans le noyau cellulaire (ADN génomique). La mesure de l'expression de ce gène sert de contrôle.

Les amorces employées sont les suivantes :

ADN 16S : sens (5'-TGG-ACA-ACC-AGC-TAT-CAC-CA-3') et anti-sens (5'-ACT-TTG-CAA-GGA-GAG-CCA-AA-3') ;
ADN COX-2 : sens (5'-AGG-CGA-CCT-GCG-ACT-CCT-TGA-3') et anti-sens (5'-TTA-GCT-TTA-CAG-TGG-GCT-CTA-GAG-GC-3') ;
ADN UCP-2 : sens (5'-CCT-AGC-GCT-GCC-TCA-TAA-AC-3') et anti-sens (5'CCT-ATG-GGT-CTG-TGC-CTG-TT-3').

Les expériences de qPCR sont menées en plaques de 96 puits. Une gamme d'ADN, correspondant au mélange d'ADN de tous les échantillons, est réalisée selon une dilution sérielle, pour chaque amorce. Les échantillons d'ADN cellulaires sont mélangés avec un mélange de MIX-PCR SYBR Green (Applied Biosystems), additionné d'eau désionisée. 45 cycles de PCR sont réalisés avec une température d'hybridation de 60°C. (Thermocycleur Stepone Plus, Applied Biosystems).

La mesure de fluorescence est effectuée au bout des 45 cycles et la quantité d'ADN obtenu est déterminée à l'aide de la gamme d'ADN.

Les résultats sont exprimés en quantité d'ADN 16S et COX2 ($Q_{COX2}$), calculée en fonction de la quantité d'ADN génomique UCP-2 ($Q_{UCP\text{-}2}$), et qui se définit comme ($Q_{COX2}$) / ($Q_{UCP\text{-}2}$).

Un rapport calculé ($Q_{COX2}$) / ($Q_{UCP\text{-}2}$) supérieur à 1 indique une augmentation de la quantité d'ADN mitochondrial, reflet d'une augmentation du nombre de mitochondries intracellulaires.

RESULTATS

Les mesures XTT après 48h de traitement montrent que les composés testés stimulent significativement les fonctions respiratoires mitochondriales des kératinocytes. Aucun effet dose significatif n'est cependant observé.

Par ailleurs, après irradiation aux $UV_B$ des kératinocytes, aucune stimulation significative des déshydrogénases n'est observée.

Les composés VT1 et VT2 sont les composés qui stimulent le plus efficacement l'activité des déshydrogénases mitochondriales de kératinocytes.

En particulier, le composé VT2 stimule significativement l'activité des déshydrogénases à la plus faible concentration testée de 0,78μg/ml soit 1μM (+39% par rapport au témoin négatif) **(fig. 6).**

Les trois composés testés activent la cytochrome C oxydase, à des niveaux de concentration plus faibles que le resvératrol (témoin positif), le composé VT2 étant le meilleur activateur du cytochrome C oxydase. A une concentration de 1,56μg/ml (2μM), VT2 stimule l'activité du cytochrome C oxydase à un niveau d'activité comparable au resvératrol à la concentration de 11,41μg/ml (50μM) **(fig.7).**

Un effet-dose est observé uniquement pour le composé VT1.

Pour VT1 et VT2, on réalise une mesure de biogenèse mitochondriale afin de savoir si l'augmentation de l'activité enzymatique mesurée est liée à une stimulation basale de la chaîne respiratoire des mitochondries initialement présentes, ou à une augmentation du nombre de mitochondries intracellulaires.

Les résultats indiquent qu'aux concentrations testées, à l'exception de VT1 à 6,25μg/ml, aucun des deux composés n'induit une activation de la biogenèse mitochondriale, donc une augmentation du nombre de mitochondries intracellulaires.

Par conséquent, les résultats obtenus, qui mettent en évidence une augmentation de l'activité enzymatique du cytochrome C oxydase ou des déshydrogénases mitochondriales, sont le reflet d'une stimulation basale de ces activités enzymatiques et non d'une augmentation du nombre de mitochondries intracellulaires.

VT1 et VT2 présentent le meilleur potentiel d'activation mitochondriale, au moyen d'une stimulation de l'activité enzymatique des déshydrogénases des complexes I et II, et par stimulation du cytochrome C oxydase de la chaîne respiratoire mitochondriale.

Les composés VT1 et plus particulièrement VT2 présentent donc chacun une activité significative vis-à-vis de la chaîne respiratoire mitochondriale de kératinocytes, par stimulation des complexes I et II de ladite chaîne et stimulation de l'activité du cytochrome C oxydase. Ceci confirme qu'ils activent le métabolisme énergétique de la cellule cutanée par l'intermédiaire de ses fonctions respiratoires mitochondriales.

Cette activité justifie l'usage des composés de l'invention, notamment VT1 et/ou VT2, en tant qu'agents actifs

cosmétiques ou dermatologiques, et plus particulièrement pour le traitement cosmétique ou dermatologique des peaux matures, tel que la lutte contre le vieillissement cutané et/ou le maintien ou l'amélioration de l'hydratation cutanée et/ou la cicatrisation cutanée.

**• Stimulation de la différenciation de kératinocytes humains normaux (KHN)**

Les desmogléines sont des cadhérines, protéines transmembranaires, qui lient les cellules épithéliales entre elles, comme les kératinocytes, grâce à la formation de desmosomes. Tout comme la loricrine, l'involucrine est une protéine exprimée par les kératinocytes constitutifs de la couche cornée et ayant atteint le stade terminal de différenciation. Ces kératinocytes sont appelés cornéocytes. Les transglutaminases, enzymes $Ca^{2+}$ dépendantes, assurent la formation de points covalents entre ces protéines. Ces trois types de protéines constituent donc des marqueurs de différenciation précoce (desmogléine I) et tardif (involucrine et transglutaminases) des kératinocytes. Un ralentissement du renouvellement des kératinocytes, de leur activité et de leur transformation en cornéocytes s'observe lors du vieillissement cutané. Une atrophie de l'épiderme est observée. La peau se déshydrate, les fonctions protectrices et de cicatrisation de l'épiderme sont alors ralenties. La peau prend alors un aspect terne et desséché.

<u>Méthode</u>

Les KHN sont cultivés en passage 3 en milieu KSFM complet en flasque T75. Au stade de pré-confluence, les KHN sont trypsinés et ensemencés à raison de 20 000 cellules par puits dans des systèmes de culture Lab-Tek II, 8 puits (Nalge Nunc International). 4 puits par condition de traitement. A 80% de confluence, les cellules sont traitées pendant 5 jours avec les différentes compositions d'actifs (VT1, VT2, VT3, chacun à 12μg/ml) jusqu'au stade de postconfluence. Le Vitactyl® clair (extrait de Malva sylvestris, Silab) à 3% est employé comme témoin positif.

<u>Immunomarquage</u>

Les cellules sont rincées au PBS (PBS Tablets, Invitrogen GIBCO) puis fixées à la formaline (Formalin Solution 10% Neutral Buffered, Sigma) pendant 10 minutes.

Après rinçage au PBS, les chambres de culture sont remplies avec une solution de Triton 0,1% (Triton X-100, Sigma) pendant 10 minutes afin de perméabiliser les membranes, puis rincées 2 fois au PBS. Les cellules sont ensuite recouvertes d'une solution de PBS/BSA 1% (BSA, Sigma) pendant 30 minutes à température ambiante. La solution de PBS/BSA est retirée des lames en les inclinant sur du papier absorbant.

Les cellules sont ensuite recouvertes d'une solution d'anticorps primaires pendant 60 minutes à température ambiante:

- anti-transglutaminase (Mouse monoclonal Harbor Bio product) diluée au 1/200ème,
- anti-desmogléine 1 (Mouse monoclonal - Zymed) diluée au 1/100ème ;
- anti-involucrine (Mouse monoclonal - Neomarkers) diluée 1/200ème.

Apres rinçage au PBS, les cellules sont ensuite recouvertes d'une solution d'anticorps secondaire dilué au $1/200^{è}$ (Alexa fluor 488, goat anti-mouse IgG (Molecular probes)). Les lames sont incubées pendant 60 minutes à température ambiante, à l'abri de la lumière. Les lames sont rincées trois fois dans du PBS.

<u>Montage</u>

La chambre de culture des lames est démontée et quelques gouttes de milieu de montage (Fluorescent Mounting Medium, DAKO) sont déposées sur les cellules puis recouvertes d'une lamelle (24 x 60 mm, Knittel Glaeser).

<u>Acquisition des photos en microscopie confocale</u>

Les images sont réalisées au vidéomicroscope (Nikon TE 2000) avec un filtre Alexa fluor 488.

Pour chaque condition, quatre photos sont réalisées en fluorescence verte (expression des marqueurs) à l'objectif x20. Les paramètres d'acquisition (temps d'exposition, gain) sont identiques pour chaque marqueur étudié.

Les photos sont analysées à l'aide du logiciel d'analyse d'image Leica QWin. Un programme est créé afin de quantifier l'expression de ces marqueurs de différenciation.

RESULTATS

Les résultats montrent que les composés de l'invention stimulent significativement l'expression des protéines de différenciation des kératinocytes, par rapport au témoin négatif.

Parmi les composés testés, VT1 et VT2 sont les plus efficaces (fig. 8 à 13). Par ailleurs VT2 stimule l'expression de l'involucrine à un niveau supérieur à celui du témoin positif employé à 3%.

Ceci démontre que les vandatérosides, et plus particulièrement VT1 et VT2, stimulent le renouvellement des kératinocytes, constitutifs de la couche supérieure de l'épiderme.

Les vandatérosides favorisent le renouvellement du *stratum corneum* donnant ainsi un aspect plus jeune à la peau. Ce phénomène contribue à maintenir les fonctions protectrices et réparatrices de l'épiderme tout en maintenant ou améliorant l'hydratation cutanée.

## Revendications

1. Composé choisi parmi le 4-(4-β-D-glucopyranosyloxybenzyl)-(2R)-2-(*p*-hydroxybenzyl)-malate (VT1), le 1,4-bis(4-β-D-glucopyranosyloxybenzyl)-(2R)-2-(*p*-hydroxybenzyl)-malate (VT2), et le 1,4-bis(4-β-D-glucopyranosyloxybenzyl)-(2R)-2-(2-β-D-glucopyranosyl-3-*trans*-cinnamoyl ester)-2-(p-hydroxybenzyl)-malate (VT3).

2. Extrait d'orchidée comprenant au moins 40% en poids d'un ou plusieurs des composés tels que définis à la revendication 1, par rapport au poids total de l'extrait sec.

3. Extrait selon la revendication 2, **caractérisé en ce qu'**il est obtenu à partir d'au moins une partie d'une orchidée appartenant au genre *Papilionanthe, Vanda* ou *Encyclia.*

4. Extrait selon la revendication 2, **caractérisé en ce qu'**il comprend un ou plusieurs composés choisis parmi VT1 et/ou VT2, à une teneur cumulée supérieure à 40% en poids par rapport au poids de l'extrait sec.

5. Extrait selon la revendication 2, **caractérisé en ce qu'**il comprend le composé VT3 à une teneur supérieure à 8% en poids par rapport au poids de l'extrait sec.

6. Extrait selon la revendication 2, **caractérisé en ce que** l'extrait est constitué par au moins 80% en poids des composés VT1, VT2 et VT3 par rapport au poids total de l'extrait sec.

7. Extrait selon l'une quelconque des revendications 2 à 6, **caractérisé en ce qu'**il est préparé à partir des tiges ou des racines de l'orchidée *Papilionanthe teres.*

8. Composition cosmétique ou dermatologique comprenant à titre d'agent actif cosmétique ou dermatologique, au moins un composé tel que défini à la revendication 1 et/ou au moins un extrait selon l'une quelconque des revendications 2 à 7.

9. Composé tel que défini à la revendication 1, ou d'au moins un extrait tel que défini à l'une quelconque des revendications 2 à 7, pour son utilisation en tant qu'agent actif cosmétique ou dermatologique dans une composition cosmétique ou dermatologique, ledit agent actif étant destiné

   - à lutter contre le vieillissement cutané, notamment pour atténuer ou retarder les effets du vieillissement de la peau, restructurer l'épiderme, raffermir la peau, et/ou favoriser l'atténuation ou la résorption des rides et les propriétés protectrices de l'épiderme, et/ou à maintenir ou améliorer l'hydratation cutanée et/ou à favoriser la cicatrisation cutanée, ou
   - à stimuler l'expression et/ou l'activité des déshydrogénases mitochondriales, et/ou de la cytochrome C oxidase, et/ou la chaîne respiratoire des mitochondries, et/ou le métabolisme énergétique des cellules, et/ou la différenciation et le renouvellement des cellules épithéliales, plus particulièrement au niveau des kératinocytes.

10. Procédé de préparation d'un composé tel que défini à la revendication 1 ou d'un extrait tel que défini à la revendication 7, **caractérisé en ce qu'**il comprend au moins une étape d'extraction réalisée à l'aide d'au moins un solvant polaire d'au moins une partie de l'orchidée *Papilionanthe teres,* comprenant un tel composé, et au moins une étape d'enrichissement en un ou plusieurs composés VT1, VT2 ou VT3 de l'extrait obtenu à l'étape d'extraction, par élimination des sucres ou par fractionnement, notamment par chromatographie d'exclusion stérique.

## Patentansprüche

1. Verbindung, die aus 4-(4-β-D-glucopyranosyloxybenzyl)-(2R)-2-(ρ-hydroxybenzyl)-malat (VT1), 1,4-bis(4-β-D-glucopyranosyloxybenzyl)-(2R)-2-(ρ-hydroxybenzyl)-malat (VT2) und 1,4-bis(4-β-D-glucopyranosyloxybenzyl)-(2R)-2-(2-β-D-glucopyranosyl-3-*trans*-cinnamoyl ester)-2-(ρ-hydroxybenzyl)-malat (VT3) ausgewählt ist.

2. Orchideen-Extrakt, umfassend wenigstens 40 Gew.-% von einer oder mehreren der Verbindungen wie sie in Anspruch 1 definiert sind, bezogen auf das Gesamtgewicht des Trockenextrakts.

3. Extrakt nach Anspruch 2, **dadurch gekennzeichnet, dass** er aus wenigstens einem Teil einer Orchidee, die zu der Gattung *Papilionanthe, Vanda* oder *Encyclia* gehört, gewonnen ist.

4. Extrakt nach Anspruch 2, **dadurch gekennzeichnet, dass** er eine oder mehrere aus VT1 und/oder VT2 ausgewählte Verbindungen in einem kumulierten Gehalt von mehr als 40 Gew.-% bezogen auf das Gewicht des Trockenextrakts umfasst.

5. Extrakt nach Anspruch 2, **dadurch gekennzeichnet, dass** er die Verbindung VT3 in einem Gehalt von mehr als 8 Gew.-% bezogen auf das Gewicht des Trockenextrakts umfasst.

6. Extrakt nach Anspruch 2, **dadurch gekennzeichnet, dass** der Extrakt durch wenigstens 80 Gew.-% der Verbindungen VT1, VT2 und VT3, bezogen auf das Gesamtgewicht des Trockenextrakts gebildet ist.

7. Extrakt nach einem der Ansprüche 2 bis 6, **dadurch gekennzeichnet, dass** er aus den Stängeln oder den Wurzeln der Orchidee *Papilionanthe teres* hergestellt ist.

8. Kosmetische oder dermatologische Zusammensetzung, die als kosmetischen oder dermatologischen Wirkstoff wenigstens eine Verbindung wie sie in Anspruch 1 definiert ist und/oder wenigstens einen Extrakt nach einem der Ansprüche 2 bis 7 umfasst.

9. Verbindung wie in Anspruch 1 definiert oder wenigstens ein Extrakt wie in einem der Ansprüche 2 bis 7 definiert, für ihre/seine Verwendung als kosmetischer oder dermatologischer Wirkstoff in einer kosmetischen oder dermatologischen Zusammensetzung, wobei der Wirkstoff dazu bestimmt ist,

   - die Hautalterung zu bekämpfen, insbesondere die Auswirkungen der Hautalterung zu mildern oder zu verzögern, die Epidermis zu restrukturieren, die Haut zu festigen und/oder die Milderung oder den Abbau der Falten und die Schutzeigenschaften der Epidermis zu begünstigen und/oder die Hydratation der Haut zu erhalten oder zu verbessern und/oder die Wundheilung der Haut zu begünstigen, oder
   - die Expression und/oder die Aktivität der mitochondrialen Dehydrogenasen und/oder des Cytochrom-c-Oxidase, und/oder die Atmungskette der Mitochondrien und/oder den Energiestoffwechsel der Zellen und/oder die Differenzierung und die Erneuerung der Epithelzellen, insbesondere im Bereich der Keratinozyten, zu stimulieren.

10. Verfahren zur Herstellung einer Verbindung wie sie in Anspruch 1 definiert ist oder eines Extraktes wie er in Anspruch 7 definiert ist, **dadurch gekennzeichnet, dass** es wenigstens einen mit Hilfe wenigstens eines polaren Lösungsmittels durchgeführten Schritt zur Extraktion wenigstens eines Teils der Orchidee *Papilionanthe teres,* umfassend eine solche Verbindung, sowie wenigstens einen Schritt zur Anreicherung des bei dem Extraktionsschritt erhaltenen Extrakts mit einer oder mehreren Verbindungen VT1, VT2 oder VT3, durch Entfernen der Zucker oder durch Fraktionierung, insbesondere durch sterische Ausschlusschromatographie, umfasst.

## Claims

1. A compound selected from the group consisting of 4-(4-β-D-glucopyranosyloxybenzyl)-(2R)-2-(*p*-hydroxybenzyl)-malate (VT1), 1,4-bis(4-β-D-glucopyranosyloxybenzyl)-(2R)-2-(*p*-hydroxybenzyl)-malate (VT2) and 1,4-bis(4-β-D-glucopyranosyloxybenzyl)-(2R)-2-(2-β-D-glucopyranosyl-3-*trans*-cinnamoyl ester)-2-(*p*-hydroxybenzyl)-malate (VT3).

2. An orchid extract comprising at least 40% by weight of one or more compounds as defined in claim 1, relative to

the total weight of the dry extract.

**3.** The extract of claim 2 wherein it is obtained from at least one part of an orchid, in particular of an orchid belonging to the *Papilionanthe, Vanda* or *Encyclia* genus.

**4.** The extract of claim 2 wherein it comprises one or more compounds chosen from VT1 and/or VT2 at a cumulative content of greater than 40% by weight, relative to the weight of the dry extract.

**5.** The extract of claim 2 wherein it comprises the VT3 compound at a content of greater than 8% by weight, relative to the weight of the dry extract.

**6.** The extract of claim 2 wherein the extract is made up of at least 80% by weight of the VT1, VT2 and VT3 compounds, relative to the total weight of the dry extract.

**7.** The extract of any one of claims 2 to 6, wherein it is obtained from stems or roots of the orchid *Papilionanthe teres.*

**8.** A cosmetic or dermatological composition comprising, as a cosmetic or dermatological active agent, at least one compound as defined in claim 1 and/or at least one extract as defined in any one of claims 2 to 7.

**9.** A compound as defined in claim 1 or at least one extract as defined in any one of claims 2 to 7 for its use as a cosmetic or dermatological active agent, in a cosmetic or dermatological composition, said active agent being aimed at:

- combating skin aging, in particular for reducing or delaying the effects of skin aging, restructuring the epidermis, firming the skin, promoting the reduction or resorption of wrinkles and the protective properties of the epidermis, and/or for maintaining or improving skin moisturization and/or for promoting skin healing, or
- stimulating the expression and/or the activity of mitochondrial dehydrogenases, and/or of cytochrome C oxidase, and/or of the mitochondrial respiratory chain, and/or cell energy metabolism and/or the differentiation and renewal of epithelial cells, more particularly in keratinocytes.

**10.** A method for preparing a compound as defined in claim 1 or an extract as defined in claim 7, wherein it comprises at least one step of extracting with at least one polar solvent at least one part of an orchid *Papilionanthe teres,* comprising such a compound, and at least one step of enriching the extract obtained in the extraction step, in one or more compounds VT1, VT2 or VT3, by removal of the sugars or by fractionation, in particular by size exclusion chromatography.

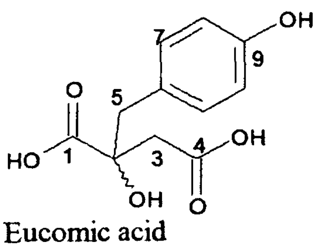

Eucomic acid

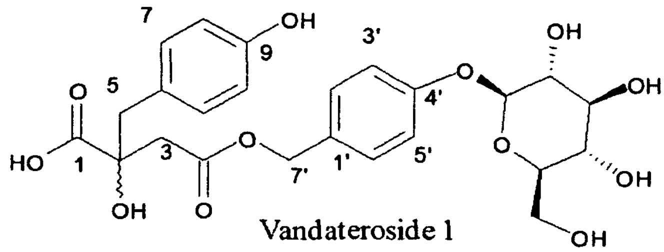

Vandateroside 1

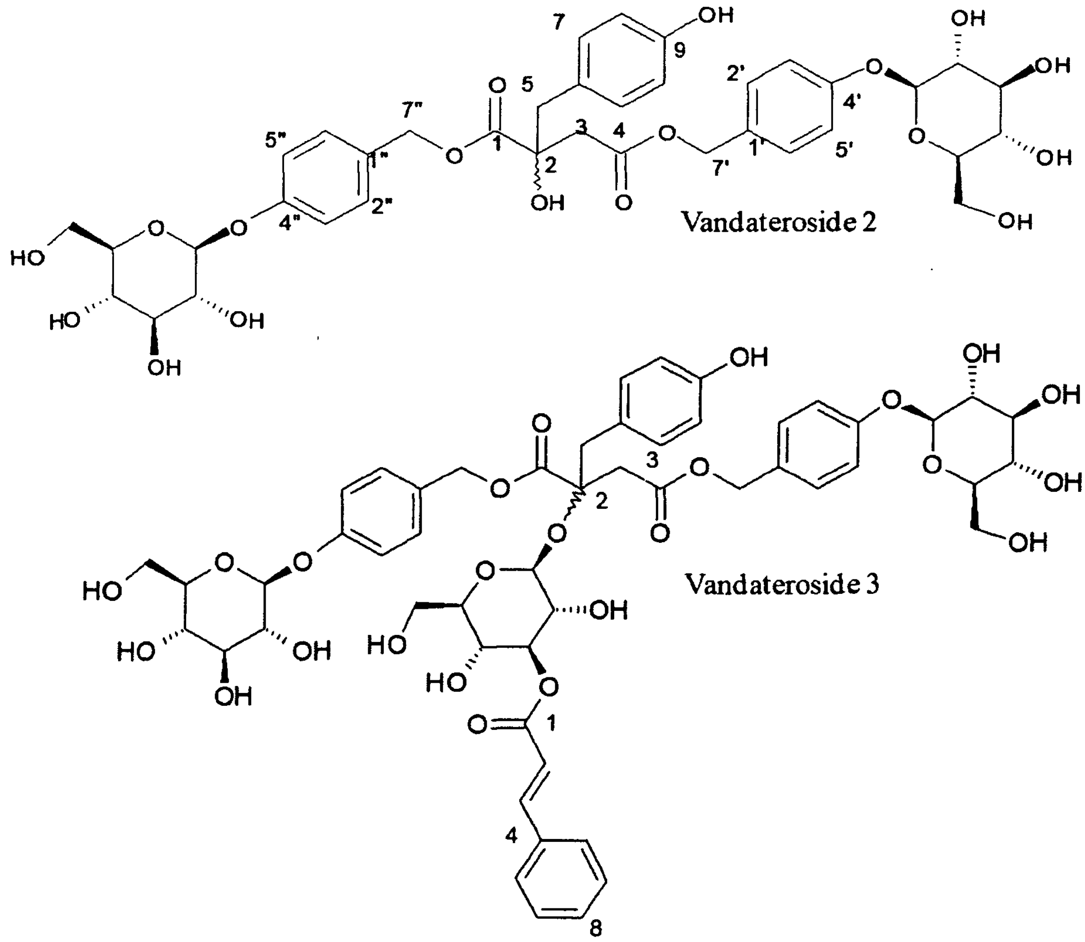

Vandateroside 2

Vandateroside 3

FIG.1

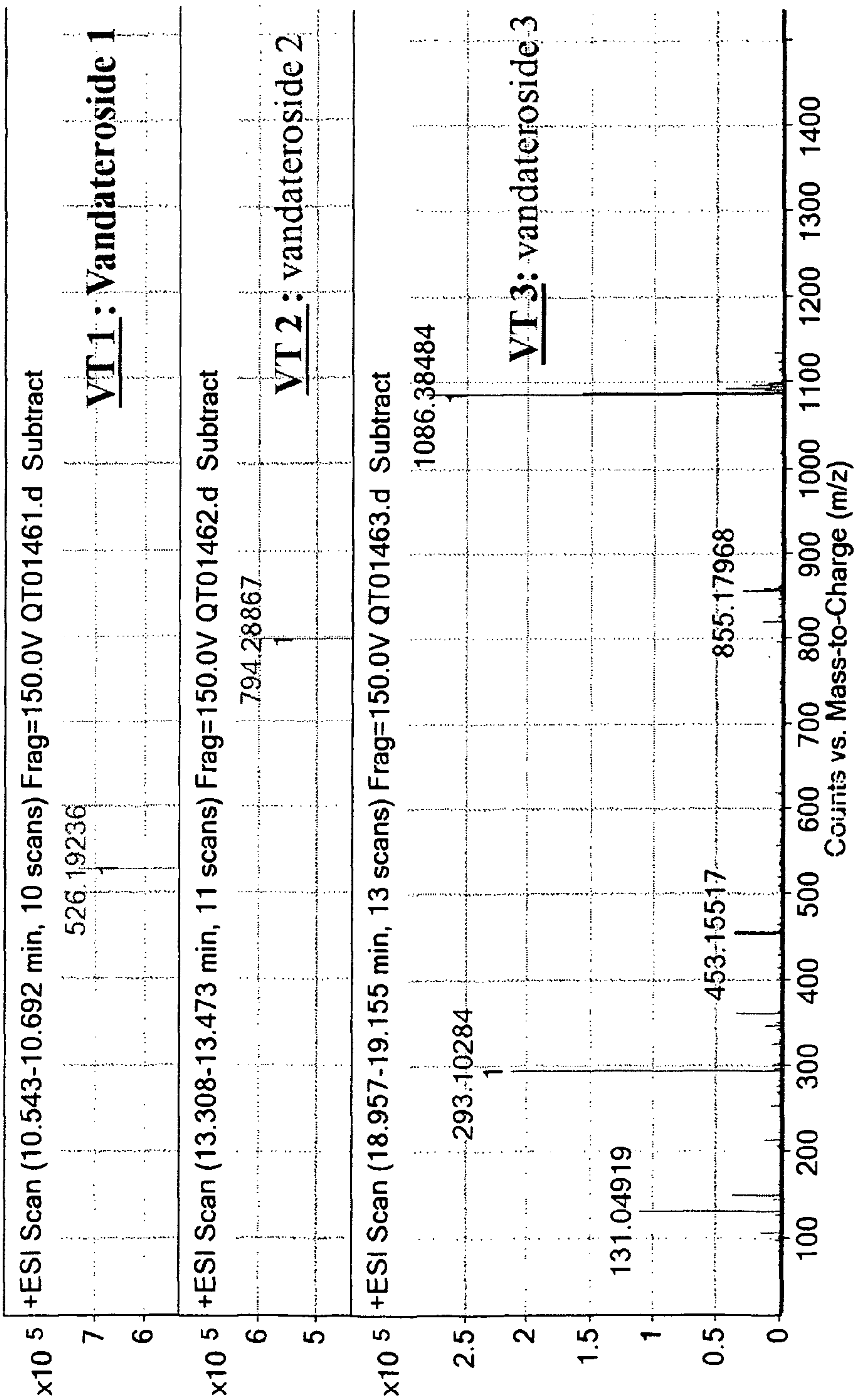
+ESI Scan (10.543-10.692 min, 10 scans) Frag=150.0V QT01461.d Subtract
VT 1 : Vandateroside 1
526.19236
+ESI Scan (13.308-13.473 min, 11 scans) Frag=150.0V QT01462.d Subtract
VT 2 : vandateroside 2
794.28867
+ESI Scan (18.957-19.155 min, 13 scans) Frag=150.0V QT01463.d Subtract
VT 3: vandateroside 3
1086.38484
855.17968
453.15517
293.10284
131.04919
x10 5
Counts vs. Mass-to-Charge (m/z)

FIG.2

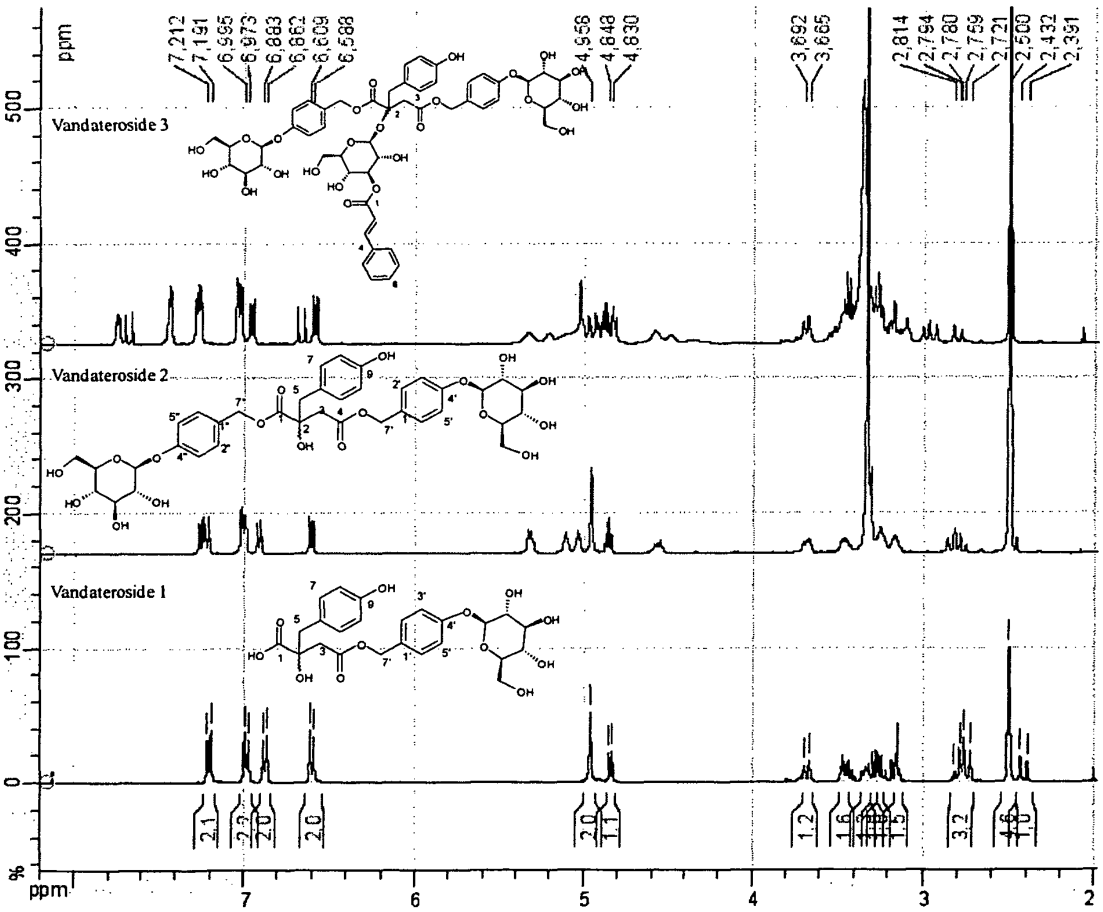
ppm
Vandateroside 3
Vandateroside 2
Vandateroside 1
7,212
7,191
6,995
6,973
6,883
6,862
6,609
6,588
4,956
4,848
4,830
3,692
3,665
2,814
2,794
2,780
2,759
2,721
2,500
2,432
2,391
%
ppm
7
6
5
4
3
2
500
400
300
200
100
0

FIG.3

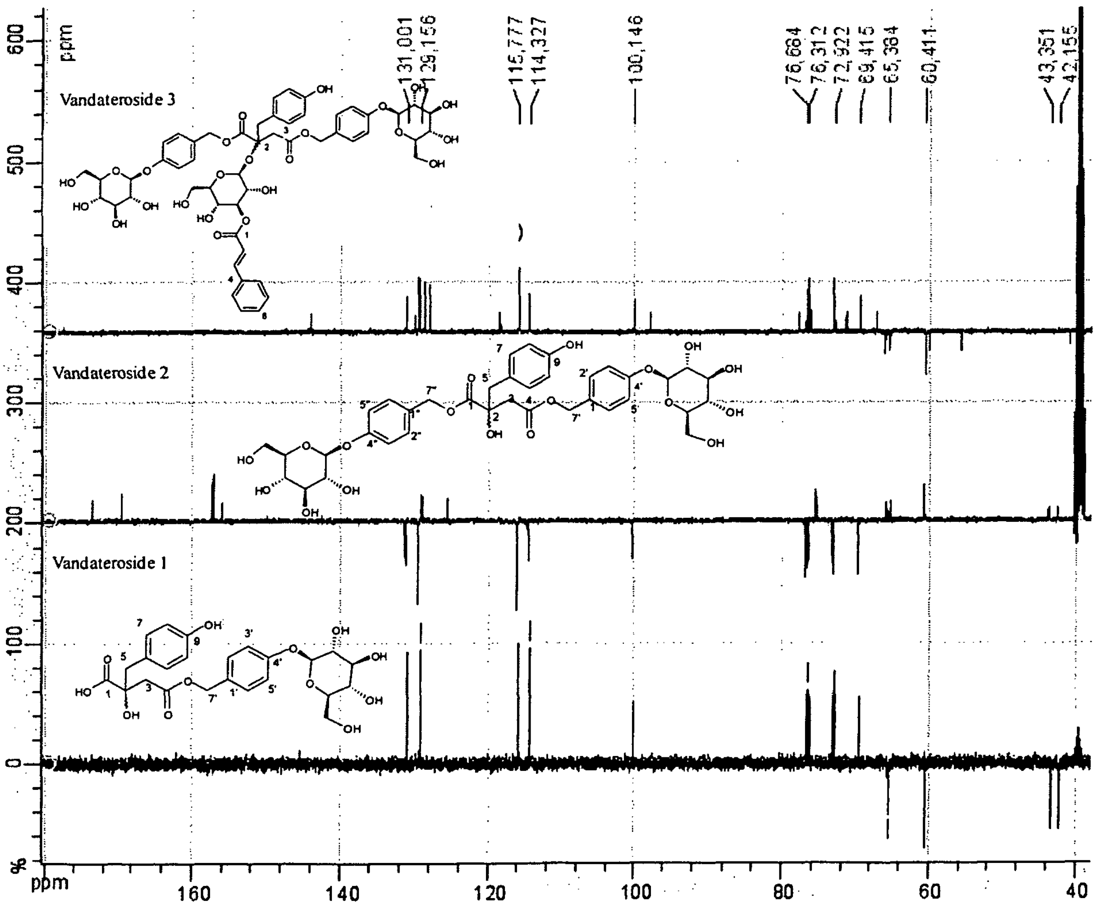
131,001
129,156
115,777
114,327
100,146
76,684
76,312
72,922
69,415
65,384
60,411
43,351
42,155
ppm
Vandateroside 3
Vandateroside 2
Vandateroside 1
600
500
400
300
200
100
0
%
ppm
160
140
120
100
80
60
40

FIG.4

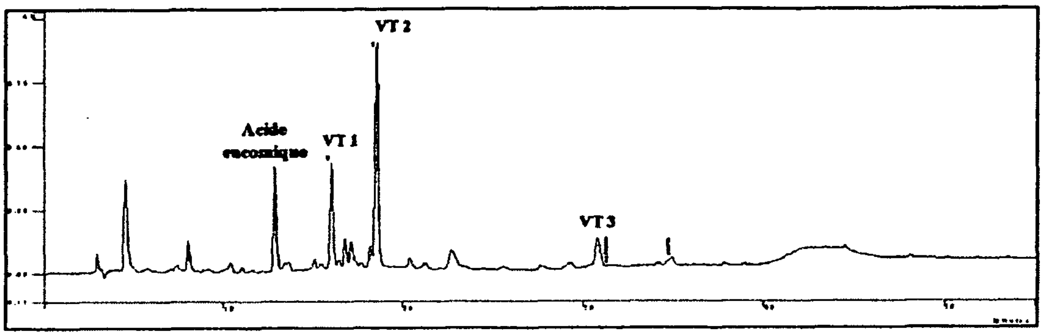
VT 2
Acide
eucomique
VT 1
VT 3

FIG.5

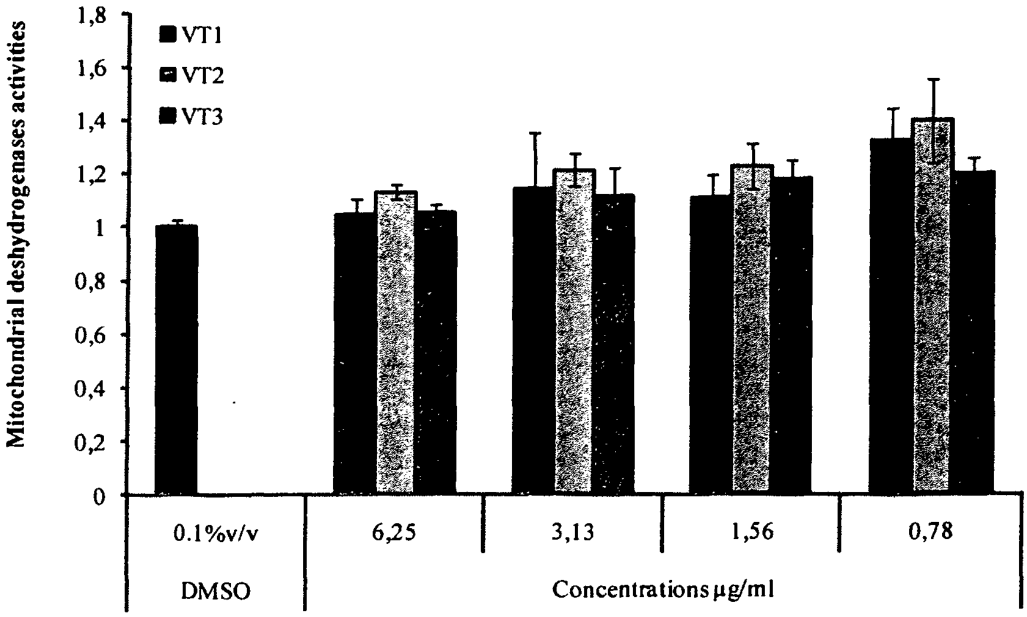
Mitochondrial deshydrogenases activities
1,8
1,6
1,4
1,2
1
0,8
0,6
0,4
0,2
0
VT1
VT2
VT3
0.1%v/v
6,25
3,13
1,56
0,78
DMSO
Concentrations µg/ml

FIG.6

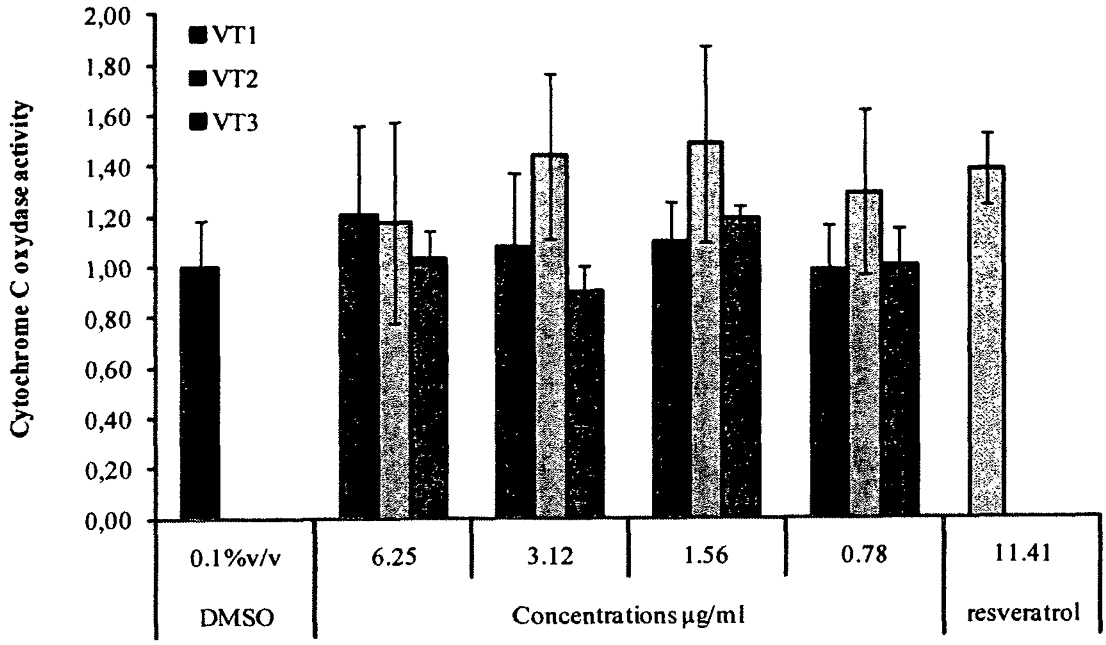
VT1
VT2
VT3
2,00
1,80
1,60
1,40
1,20
1,00
0,80
0,60
0,40
0,20
0,00
Cytochrome C oxydase activity
0.1%v/v
6.25
3.12
1.56
0.78
11.41
DMSO
Concentrations µg/ml
resveratrol

FIG.7

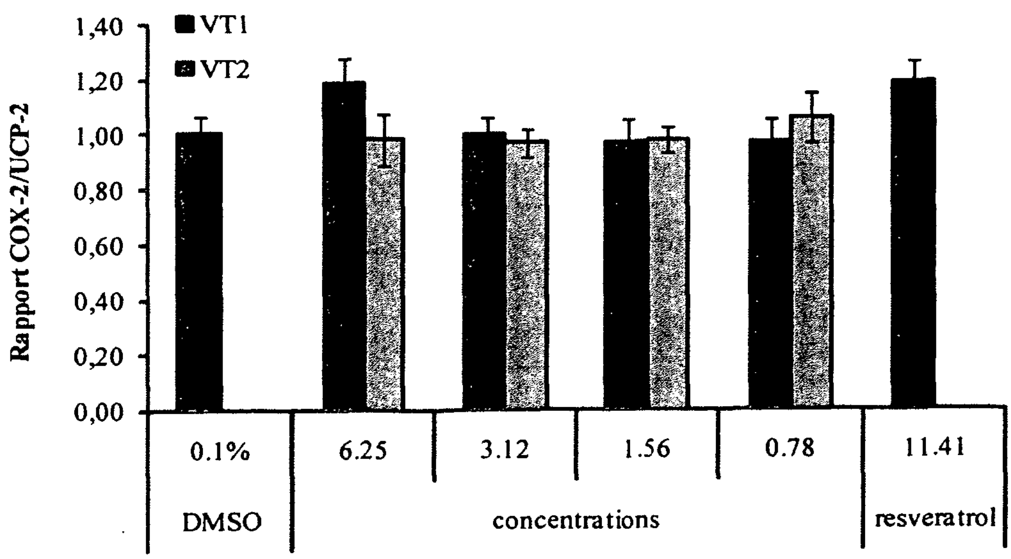
VT1
VT2
1,40
1,20
1,00
0,80
0,60
0,40
0,20
0,00
Rapport COX-2/UCP-2
0.1%
6.25
3.12
1.56
0.78
11.41
DMSO
concentrations
resveratrol

FIG.8

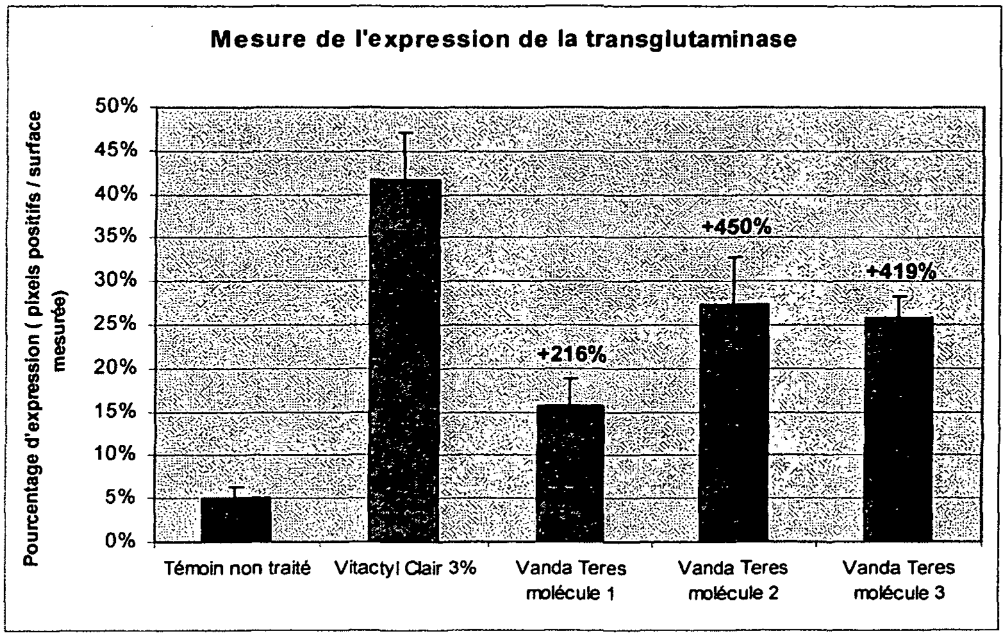
Mesure de l'expression de la transglutaminase
Pourcentage d'expression ( pixels positifs / surface mesurée)
50%
45%
40%
35%
30%
25%
20%
15%
10%
5%
0%
+216%
+450%
+419%
Témoin non traité
Vitactyl Clair 3%
Vanda Teres molécule 1
Vanda Teres molécule 2
Vanda Teres molécule 3

FIG.9

Mesure de l'expression de la transglutaminase

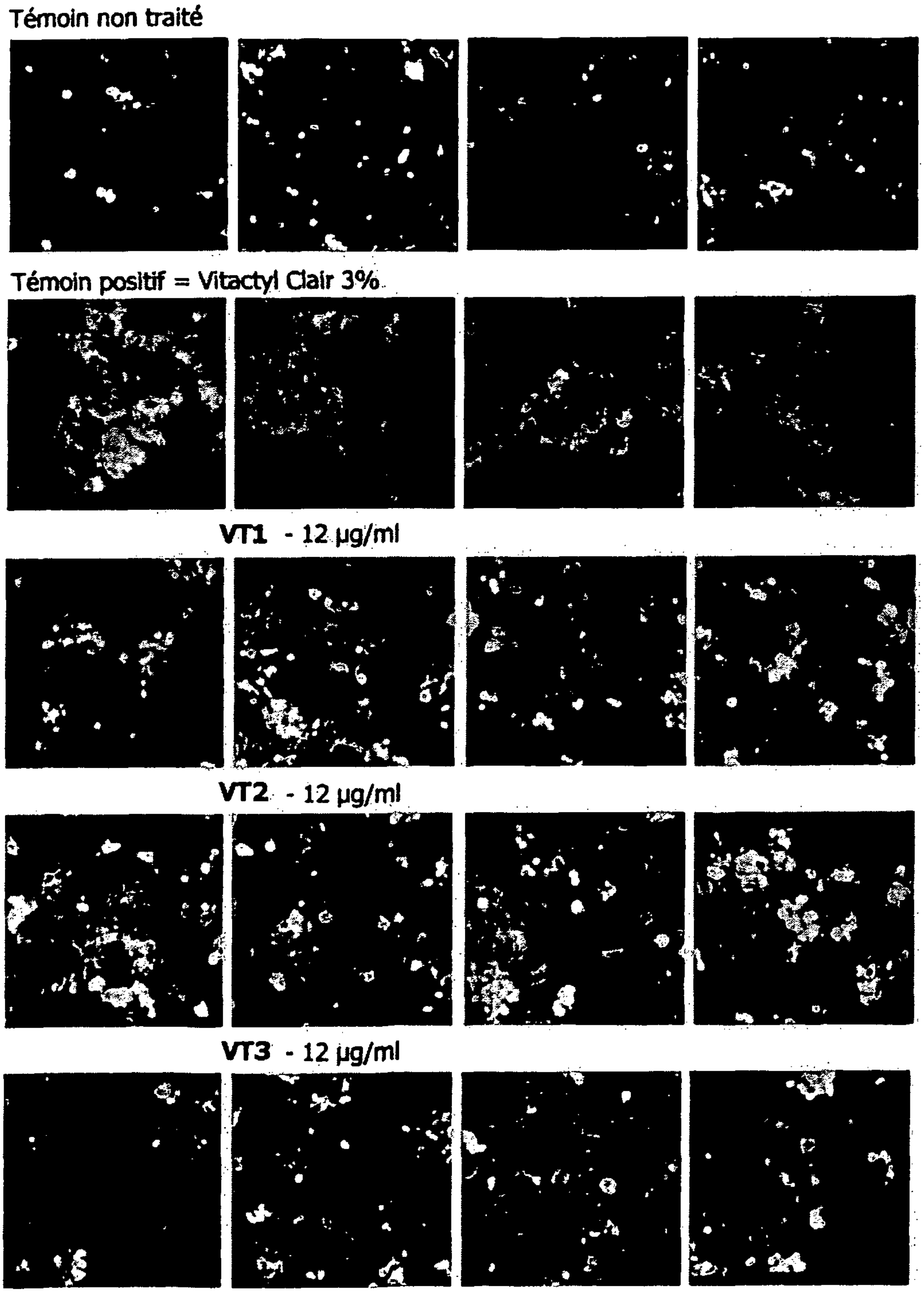


FIG.10

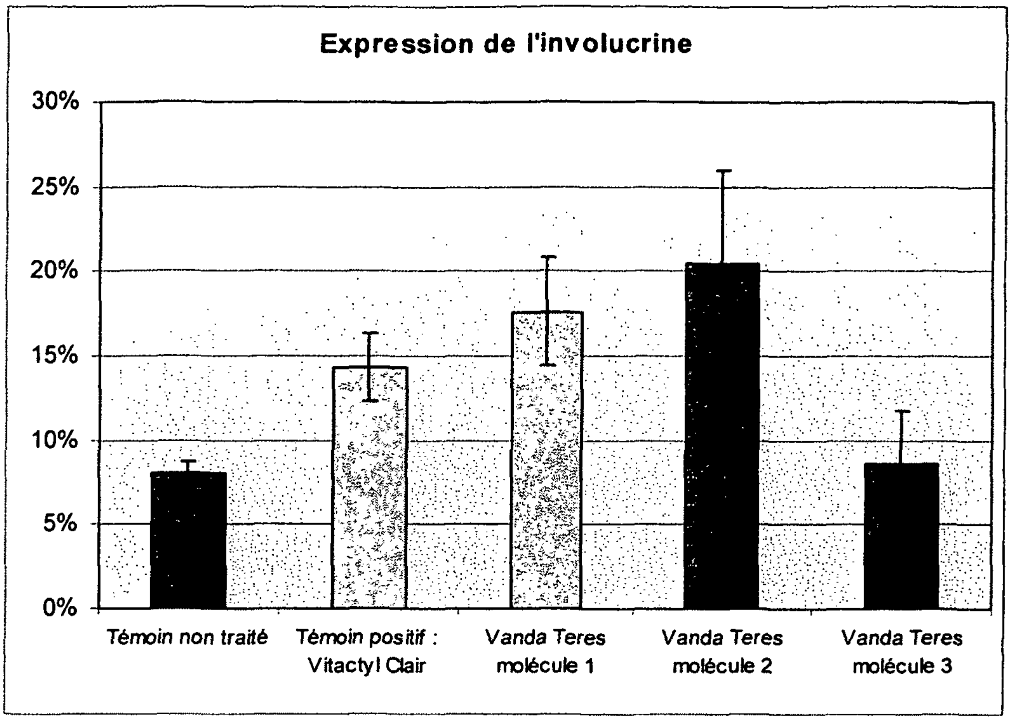
Expression de l'involucrine
30%
25%
20%
15%
10%
5%
0%
Témoin non traité
Témoin positif : Vitactyl Clair
Vanda Teres molécule 1
Vanda Teres molécule 2
Vanda Teres molécule 3

FIG.11

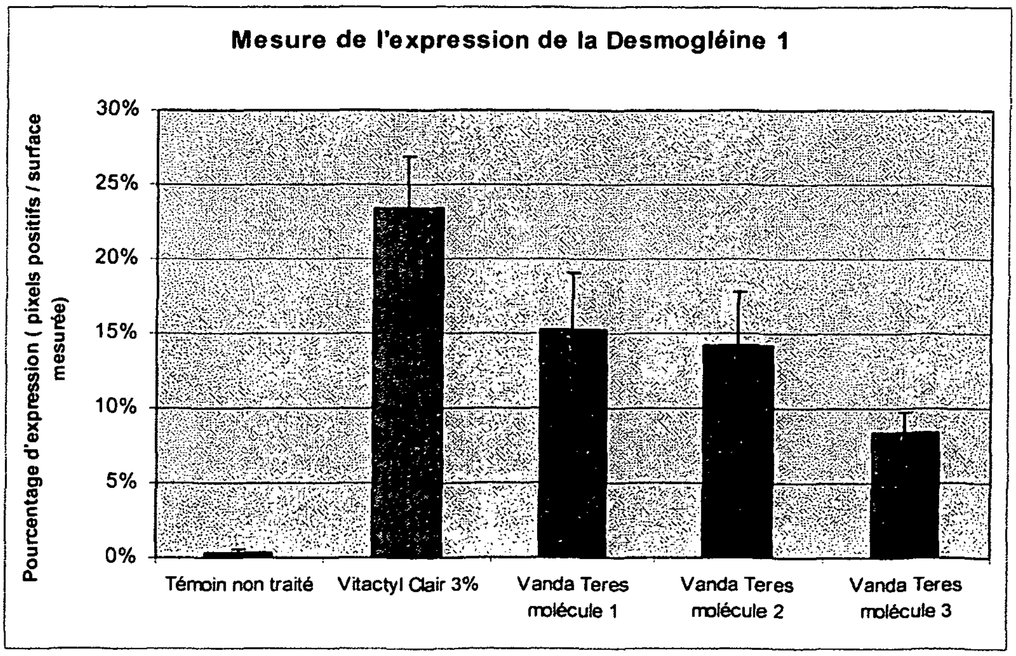
Mesure de l'expression de la Desmogléine 1
Pourcentage d'expression ( pixels positifs / surface mesurée)
30%
25%
20%
15%
10%
5%
0%
Témoin non traité
Vitactyl Clair 3%
Vanda Teres molécule 1
Vanda Teres molécule 2
Vanda Teres molécule 3

FIG.12

Mesure de l'expression de la desmogléine 1

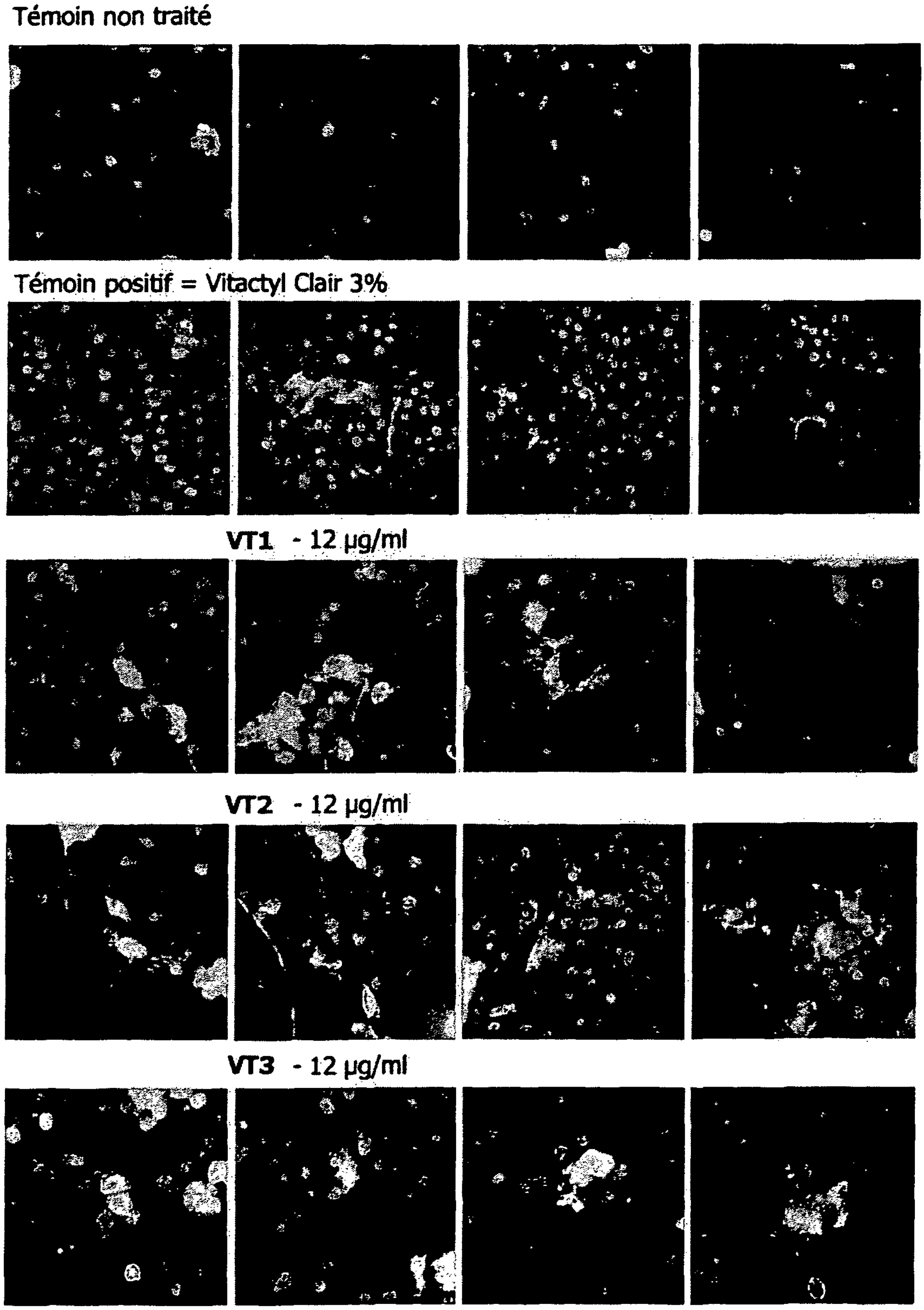


FIG.13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2924348


### Littérature non-brevet citée dans la description


- **HELLER et al.** *Helvetica Chimica Acta,* 1984, vol. 57 (6), 1766-84
- **ROEHM et al.** *J. Immunol. Methods,* 1991, vol. 142, 257-265